# EUROPEAN PATENT APPLICATION

(11) **EP 2 311 841 A1**
(43) Date of publication of application: **20.04.2011**
(21) Application number: 09742185.3
(22) Date of filing: 05.05.2009
(51) Int. Cl.: C07D 493/08, C07D 493/10, C07D 307/20, C07D 317/30, C07C 69/60, C07D 303/40

(54) **METHOD FOR OBTAINING ZARAGOZIC ACID AND DERIVATIVES THEREOF**

(30) Priority: 06.05.2008 ES 200801303
(71) Applicant: Consejo Superior De Investigaciones Científicas, 28006 Madrid (ES)
(72) Inventor: NOHEDA MARÍN, Pedro, E-28006 Madrid (ES); LOZANO GORDILLO, Luis Miguel, E-28050 Madrid (ES)
(74) Representative: ABG Patentes, S.L.
(86) International application number: PCT/ES2009/070140
(87) International publication number: WO 2009/135979

(57) **Abstract**

The present invention is aimed at a process for obtaining zaragozic acid and derivatives thereof, to the intermediate compounds of this synthesis and to the use of these intermediate compounds in the preparation of zaragozic acid.

## Description

### Field of the Invention

The present invention relates to a process for the synthesis of zaragozic acid and derivatives thereof, and to intermediates of said synthesis. It also relates to the use of said intermediates in the synthesis of zaragozic acid.

### Background of the Invention

Zaragozic acids [Nadin, A.; Nicolaou, K. C. Angew. Chem. Int. Ed. Engl. 1996, 35, 1622-1656] are a family of natural products, of which the first to be isolated was zaragozic acid A (**IA**, Scheme 1). It was simultaneously isolated between the years 1991 and 1992 by three independent groups: the Merck Group, which called it zaragozic acid A, whereas the Glaxo and Tokyo Noko University/Mitsubishi Kasei Corporation groups called it squalestatin S 1.

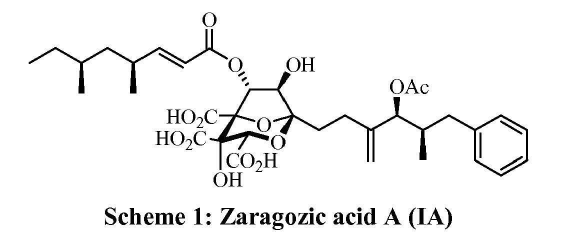

WO 93/16066 and WO 93/17557 describe the isolation of derivatives of zaragozic acids from different fungi cultures. Likewise, the chemical modification of the compounds obtained and their usefulness as cholesterol level-reducing agents are shown. WO 94/04144 also discloses various analogs of zaragozic acids, as well as their squalene synthase enzyme inhibitory activity.

Zaragozic acids have in their structure a common bicyclic grouping, 2,8-dioxabicyclo[3.2.1]octane (rings ***A*** and ***B*,** Scheme 1), which has 6 consecutive stereocenters (carbons C3, C4, C5, C6, C7 and C1), three of which are quaternary (carbons C1, C4 and C5). The structural differences between the members of the family of zaragozic acids lies in the different R_{y} and Rₓ substituents (see Scheme 2) that they have in the C6 and C1 positions, respectively, of the common bicyclic backbone.

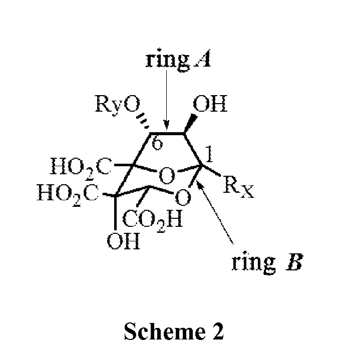

Due to their significant biological activity, as agents useful in cholesterol reduction, and to their high structural complexity, zaragozic acids have caught the attention of a number of research groups. Three total syntheses of zaragozic acid A **(IA)** have been described in the literature to date. One was developed by Dr. Nicolaou [a) Nicolaou, K. C.; Yue, E. W.; Naniwa, Y.; De Riccardis, F.; Nadin, A.; Leresche, J. E.; La Greca, S.; Yang, Z. Angew. Chem. Int. Ed. Engl. 1994, 33, 2184-2187; b) Nicolaou, K. C.; Nadin, A.; Leresche, J. E.; La Greca, S.; Tsuri, T.; Yue, E. W.; Yang, Z. Angew. Chem. Int. Ed. Engl. 1994, 33, 2187-2190; c) Nicolaou, K. C.; Nadin, A.; Leresche, J. E.; Yue, E. W.; La Greca, S. Angew. Chem. Int. Ed. Engl. 1994, 33, 2190-2191; d) Nicolaou, K. C.; Yue, E. W.; La Greca, S.; Nadin, A.; Yang, Z.; Leresche, J. E.; Tsuri, T.; Naniwa, Y. De Riccardis, F. Chem. Eur. J. 1995, 1, 467-494; e) Nicolaou, K. C.; Sorensen, E. J. Classics in Total Synthesis; VCH Publishers: New York, 1996. pp.: 673-709.], another one by Dr. Heathcock [a) Stoermer, D.; Caron, S.; Heathcock, C. H. J. Org. Chem. 1996, 61, 9115-9125; b) Caron, S.; Stoermer, D.; Mapp, A. K.; Heathcock, C. H. J. Org. Chem. 1996, 61, 9126-9134]; and one by Dr. Tomooka [Tomooka, K.; Kikuchi, M.; Igawa, K.; Suzuki, M.; Keong, P. -H.; Nakai, T. Angew. Chem. Int. Ed. 2000, 39, 4502-4505]. Five total syntheses have been developed for zaragozic acid C (**IC**), by the groups of Dr. Carreira, [a) Carreira, E. M.; Du Bois, J. J. Am. Chem. Soc. 1994, 116, 10825-10826; b) Carreira, E. M.; Du Bois, J. J. Am. Chem. Soc. 1995, 117, 8106-8125], Dr. Evans, [Evans, D. A.; Barrow, J. C.; Leighton, J. L.; Robichaud, A. J.; Sefkow, M. J. Am. Chem. Soc. 1994, 116, 12111-12112], Dr. Armstrong [a) Armstrong, A.; Jones, L. H.; Barsanti, P. A. Tetrahedron Lett. 1998, 39, 3337-3340; b) Armstrong, A.; Barsanti, P. A.; Jones, L. H.; Ahmed, G. J. Org. Chem. 2000, 65, 7020-7032] and two by Dr. Hashimoto [a) Kataoka, O.; Kitagaki, S.; Watanabe, N.; Kobayashi, J.; Nakamura, S.; Shiro, M.; Hashimoto, S. Tetrahedron Lett. 1998, 39, 2371-2374; b) Nakamura, S.; Hirata, Y.; Kurosaki, T.; Anada, M.; Kataoka, O.; Kitagaki, S.; Hashimoto, S. Angew. Chem. Int. Ed. 2003, 42, 5351-5355; a) Sato, H.; Nakamura, S.; Watanabe, N.; Hashimoto, S. Synlett 1997, 451-454; b) Nakamura, S.; Sato, H.; Hirata, Y.; Watanabe, N.; Hashimoto, S. Tetrahedron 2005, 61, 11078-11106; c) Nakamura, S. Chem. Pharm. Bull. 2005, 53, 1-10]).

On the other hand, the Johnson group prepared the common backbone of zaragozic acids from cycloheptatriene [Xu, Y.; Johnson, C. R. Tetrahedron Lett. 1997, 38, 1117-1120].

All of them have drawbacks when they are applied due to the large number of steps that they require, the low yield and the large number of protecting groups that they require. It is therefore necessary to provide a process for obtaining zaragozic acid and derivatives thereof, which preferably meets the following characteristics:
- using a small number of protecting groups;
- feasible and inexpensive commercial starting substrates;
- a reasonable number of steps;
- non-sophisticated experimental processes; or
- a good final yield.

### Summary of the Invention

It has now been found that by following the synthetic sequence of the invention it is possible to obtain compounds of formula (I) from compounds of formula (II) or (III) in a reduced number of steps and with a high yield. Said compounds of formula (I) are versatile intermediates in the preparation of zaragozic acid and derivatives thereof of formula (XXVI), because they contain the bicyclic structure 2,8-dioxabicyclo[3.2.1]octane, common to all zaragozic acids, which allows, through this common structure, obtaining the different derivatives of known zaragozic acid.

Therefore, a first aspect of the present invention is aimed at a process for obtaining a compound of formula (I), its enantiomers or mixtures thereof, **characterized in that** it comprises reacting in acidic medium a compound of formula (II), its enantiomers or mixtures thereof, or a compound of formula (III), its enantiomers or mixtures thereof, or a mixture of compounds of formula (II) and (III).

Additional aspects of the invention are aimed at compounds of formula (I), (II), (III), (IV), (V), (VI), (VII), (VIIa), (VIIb), (VIII), (VIIa), (VIIIb), (IX), (IXa), (IXb) (X), (XI), (XII), (XIII), (XIV) and (XV), their stereoisomers, especially enantiomers, or mixtures thereof, as well as to processes for obtaining them.

An additional aspect of the present invention is aimed at a process for preparing zaragozic acid and derivatives thereof of formula (XXVI), their stereoisomers, especially enantiomers, or mixtures thereof, **characterized in that** it comprises the following steps
(i) reacting in acidic medium a compound of formula (II), its enantiomers or mixtures thereof, or a compound of formula (III), its enantiomers or mixtures thereof, or a mixture of compounds of formula (II) and (III), to obtain a compound of formula (I), its enantiomers or mixtures thereof;
(ii) hydrolyzing in basic medium the ester groups of said compound of formula (I), its enantiomers or mixtures thereof, to provide a compound of formula (XXV), its enantiomers or mixtures thereof; and
(iii) reacting said compound of formula (XXV), its enantiomers or mixtures thereof, with a compound of formula (XXII) in the presence of a base, to obtain a compound of formula (XXVI), its enantiomers or mixtures thereof.

An additional aspect of the present invention is aimed at the use of a compound of formula (I), (II), (III), (IV), (V), (VI), (VII), (VIIa), (VIIb), (VIII), (VIIa), (VIIIb), (IX), (IXa), (IXb), (X), (XI), (XII), (XIII), (XIV), (XV) and/or (XVI), its stereoisomers, especially enantiomers, or mixtures thereof, for the synthesis of zaragozic acid and derivatives thereof, of formula (XXVI), their stereoisomers, especially enantiomers, or mixtures thereof.

### Detailed Description of the Invention

A first aspect of the present invention is aimed at a process for obtaining a compound of formula (I), its enantiomers or mixtures thereof 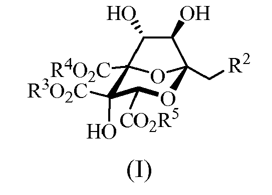 wherein
R² is selected from the group consisting of C₁-C₂₀ alkyl and C₁-C₂₀ alkenyl, which are unsubstituted or substituted in any position with at least one group which is selected from the group consisting of C₁-C₄ alkyl, C₁-C₃ alkylidene, C₁-C₃ alkylcarboxyhydroxyl, hydroxyl and protected hydroxyl; and/or substituted with a group in the end position of the chain which is selected from the group consisting of C₆-C₁₀ aryl, mono- or bicyclic heteroaryl with 5- or 6-members in each ring, which can be unsubstituted or substituted with at least one group which is selected from the group formed by C₁-C₃ alkyl, or halogen; and
R³, R⁴ and R⁵ are independently selected from the group of C₁-C₃ alkyls; **characterized in that** it comprises reacting in acidic medium a compound of formula (II), its enantiomers or mixtures thereof, or a compound of formula (III), its enantiomers or mixtures thereof, or a mixture of compounds of formula (II) and (III) 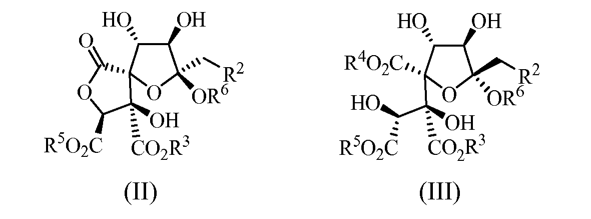 wherein
R², R³, R⁴ and R⁵ are as defined above; and
R⁶ is a C₁-C₃ alkyl group.

According to a preferred embodiment, R³ and R⁴ are identical, more preferably R³, R⁴ and R⁵ are identical, preferably methyl. The compounds of formula (II) and (III) already contain all the stereocenters of zaragozic acid and its derivatives. Without wishing to be bound by theory, it seems that first the OR⁶ group is lost with the simultaneous or subsequent formation of an oxonium ion and subsequent rearrangement to form a compound of formula (I). Therefore, the formation of the compounds of formula (I) is independent of the stereochemistry in the acetalic position of the tetrahydrofuran ring of the compounds of formula (II) and (III).

According to a preferred embodiment, the acidic medium comprises the addition of an inorganic protic acid, for example, HCl, H₂SO₄ or HNO₃. The acidic medium is preferably a diluted acidic medium, preferably at a concentration in volume with respect to the total volume of the reaction comprised between 0.1 and 20%, more preferably between 0.5 and 10%, more preferably between 1 and 5%. According to another preferred embodiment, the solvent is an alcohol of formula R⁶OH. According to another preferred embodiment, the reaction is performed at a temperature comprised between 0 and 100°C, more preferably between 25 and 90°C, more preferably between 50 and 80°C. The reaction is preferably performed in a closed vessel (for example, a Kimble ®).

Additional aspects of the present invention are the compounds of formula (II) and of formula (III), their enantiomers or mixtures thereof.

An additional aspect of the present invention is aimed at a process for the synthesis of a compound of formula (II), its enantiomers or mixtures thereof, or of a compound of formula (III), its enantiomers or mixtures thereof, **characterized in that** it comprises the dihydroxylation of a compound of formula (IV), its enantiomers or mixtures thereof 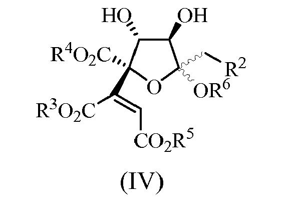 wherein
R², R³, R⁴ and R⁵ are as defined above; and
R⁶ is a C₁-C₃ alkyl group.

Therefore, in two synthetic steps from a compound of formula (IV), the creation of the nucleus of zaragozic acid and its derivatives, by means of simple reactions (dihydroxylation and acidic medium) and a high or acceptable yield, and with the additional advantage of not needing additional protecting groups, has been achieved. The dihydroxylation of the compounds of formula (IV) proceeds, followed in the same reaction medium by a rearrangement without needing to form the diol in a previous step, its subsequent protection, and subsequent deprotection prior to the cyclization.

The dihydroxylation reaction is a reaction widely used in the synthesis of organic molecules and can be performed under conditions known by the skilled person, as described in Smith, M. B.; March, J. March's Advanced Organic Chemistry; John Wiley & Sons: New York, 2001. pp.: 1048-1051. According to a preferred embodiment, the dihydroxylation is performed in the presence of osmium tetroxide/*N* methylmorpholine-N-oxide or potassium permanganate. More preferably, the hydroxylation is performed in the presence of RuCl₃/NaIO₄ (for conditions useful for performing this transformation, see a) Shing, T. K. M.; Tai, V. W.-F.; Tam, E. K. W. Angew. Chem. Int. Ed. Engl. 1994, 33, 2312-2313; b) Shing, T. K. M.; Tai, V. W.-F.; Tam, E. K. W.; Chung, I. H. F.; Jiang, Q. Chem. Eur. J. 1996, 2, 50-57; or c) Plietker, B.; Niggemann, M. Org. Lett. 2003, 5, 3353-3356).

According to a preferred embodiment, the process comprises dihydroxylating in the presence of RuCl₃ a compound of formula (IVa), its enantiomers or mixtures thereof, to yield a compound of formula (II), its enantiomers or mixtures thereof 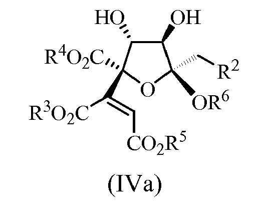 wherein
R², R³, R⁴ and R⁵ are as defined above; and
R⁶ is a C₁-C₃ alkyl group.

According to another preferred embodiment, the process comprises dihydroxylating in the presence of RuCl₃ a compound of formula (IVb), its enantiomers or mixtures thereof, to yield a compound of formula (III), its enantiomers or mixtures thereof 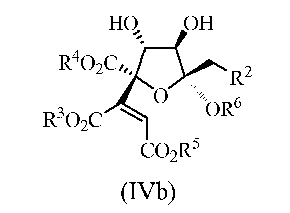 wherein
R², R³, R⁴ and R⁵ are as defined above; and
R⁶ is a C₁-C₃ alkyl group.

Therefore, another aspect of the present invention is aimed at compounds of formula (IV), (IVa) or (IVb) their stereoisomers, especially their enantiomers, or mixtures thereof as defined above, which are intermediates which allow obtaining the compounds of formula (I), their enantiomers or mixtures thereof, and therefore, also zaragozic acid and its derivatives of formula (XXVI).

An additional aspect of the present invention is aimed at a process for the synthesis of a compound of formula (IV), its stereoisomers, especially its enantiomers, or mixtures thereof, comprising the treatment in acidic medium of a compound of formula (V), its stereoisomers, especially its enantiomers, or mixtures thereof 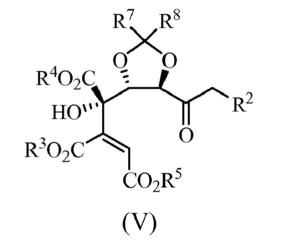 wherein
R², R³, R⁴ and R⁵ are as defined above; and
each of R⁷ and R⁸ is independently selected from the group consisting of hydrogen, C₁-C₄ alkyl and C₆-C₁₀ aryl; or R⁷ and R⁸, together with the carbon atom to which they are attached, form a C₂-C₇ alkylidene group.

The conditions under which this process is performed are the typical ones for the elimination of acetals, which in this case is surprisingly accompanied by the concomitant cyclization to form the acetalic tetrahydrofuran ring characteristic of the compounds of formula (IV). Conditions useful for obtaining this transformation can be found in a) Lu, W.; Zheng, G.; Cai, J. Tetrahedron 1999, 55, 4649-4654; or b) Greene, T. W.; Wuts, P. G. M. Greene's Protective Groups in Organic Synthesis; John Wiley & Sons: Hoboken, 2007. pp.: 306-321, which are incorporated by reference. According to a preferred embodiment, the reaction is performed in the presence of para-toluenesulfonic acid (p-TsOH). According to another preferred embodiment, the solvent is an alcohol of formula R⁶OH more preferably also of formula R⁴OH.

Therefore, another aspect of the present invention is aimed at a compound of formula (V) its stereoisomers, especially its enantiomers, or mixtures thereof as defined above, intermediates useful for the synthesis of zaragozic acid and its derivatives of formula (XXVI).

An additional aspect of the present invention relates to a process for the synthesis of a compound of formula (V), its stereoisomers, especially its enantiomers, or mixtures thereof, **characterized in that** it comprises
(i) the removal of the trialkylsilyl group of a compound of formula (VII), its stereoisomers, especially its enantiomers, or mixtures thereof, to yield a compound of formula (VI), its stereoisomers, especially its enantiomers, or mixtures thereof 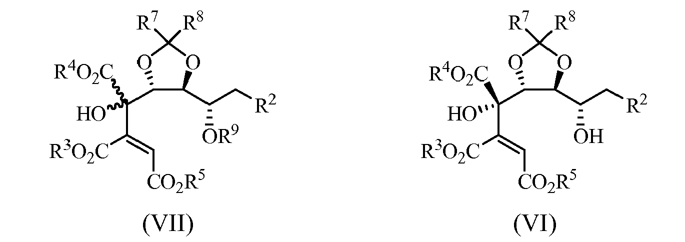 wherein
   R², R³, R⁵, R⁴, R⁷ and R⁸ are as defined above; and
   R⁹ is a trialkylsilyl group; and
(ii) the oxidation of the hydroxyl group of a compound of formula (VI), its stereoisomers, especially its enantiomers, or mixtures thereof.

As can be observed, step (i) comprises the removal of the trialkylsilyl group, i.e., the transformation of R⁹ into a hydrogen. Said transformation is normally understood as a deprotection and can be performed under different conditions (for example, see Kocienski, P. J. Protecting Groups; Thieme: Stuttgart, 2000. pp.: 188-230). According to a preferred embodiment, the trialkylsilyl group is removed from a compound of formula (VII) to give rise to a compound of formula (VI) in diluted acidic medium, such as 1% HCl for example.

According to a preferred embodiment, step (ii) is performed in the presence of PCC or IBX, preferably IBX. Suitable conditions are described in a) Frigerio, M.; Santagostino, M.; Sputore, S.; Palmisano, G. J. Org. Chem. 1995, 60, 7272-7276; b) Frigerio, M.; Santagostino, M. Tetrahedron Lett. 1994, 35, 8019-8022; c) Corey, E. J.; Palani, A. Tetrahedron Lett. 1995, 36, 3485-3488; d) Wirth, T. Angew. Chem. Int. Ed. 2001, 40, 2812-2814. Preferably, ethyl acetate is used as a solvent and in the workup of the reaction the excess reagent, as well as the derivative byproducts thereof, can be removed by means of filtration once the reaction has concluded. (More, J. D.; Finney, N. S. Org. Lett. 2002, 4, 3001-3003).

Therefore, another aspect of the present invention is aimed at compounds of formula (VI) or (VII), their stereoisomers, especially their enantiomers, or mixtures thereof as defined above, which are intermediates which allow obtaining zaragozic acid and its derivatives of formula (XXVI).

An additional aspect of the present invention is aimed at a process for the synthesis of a compound of formula (VII), its stereoisomers, especially its enantiomers, or mixtures thereof, which comprises reacting a compound of formula (VIII), its enantiomers, or mixtures thereof, with a compound of formula (XX) 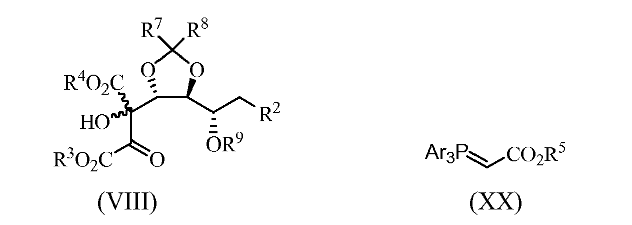 wherein
R², R³, R⁵, R⁴, R⁷, R⁸ and R⁹ are as defined above; and
each of the Ar groups is independently selected from among C₆-C₁₀ aryl groups.

The preparation of the ylide of formula (XX) can be performed according to conditions known in the state of the art (Villa, M. J.; Warren, S. J. Chem. Soc. P. T 1 1994, 12, 1569-1572) or be commercially purchased. According to a preferred embodiment, said ylide is [(methoxycarbonyl)methylene]triphenylphosphorane.

Both the compounds of formula (VII) and the compounds of formula (VIII) are useful for obtaining zaragozic acid independently of the configuration of carbon C7. The configuration of said tertiary hydroxyl can be inverted, for example, according to the conditions described in Shi, Y. -J.; Hughes, D.L.; McNamara, J.M. Tetrahedron Lett. 2003, 44, 3609-3611; or Mukaiyama, T.; Shintou, T.; Fukumto, K. J. Am. Chem. Soc. 2003, 125, 10538-10539). Therefore, according to a preferred embodiment, the compounds of formula (VII), their stereoisomers, especially their enantiomers, or mixtures thereof, are compounds of formula (VIIa) or (VIIb), their stereoisomers, especially their enantiomers, or mixtures thereof

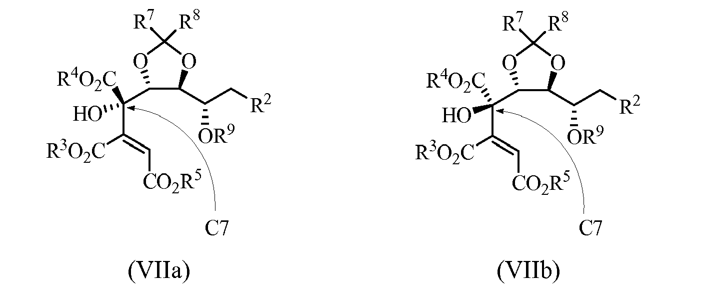

According to another preferred embodiment, the compounds of formula (VIII), their stereoisomers, especially their enantiomers, or mixtures thereof, are compounds of formula (VIIIa) or (VIIIb), their stereoisomers, especially their enantiomers, or mixtures thereof

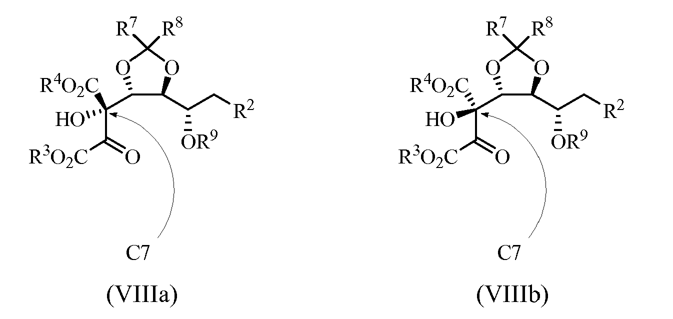

According to a preferred embodiment, a compound of formula (VIIb), its stereoisomers, especially its enantiomers, or mixtures thereof, is transformed into a compound of formula (VIIa), its stereoisomers, especially its enantiomers, or mixtures thereof, by means of a Mitsunobu configuration inversion reaction. Said transformation is preferably performed under the conditions described in Mukaiyama, T.; Shintou, T.; Fukumto, K. J. Am. Chem. Soc. 2003, 125, 10538-10539, which is incorporated in its entirety by reference, more preferably in the presence of another chlorodiphenylphosphine and a carboxylic acid. According to another preferred embodiment, said transformation is performed under the conditions described in Shi, Y. -J.; Hughes, D.L.; McNamara, J.M. Tetrahedron Lett. 2003, 44, 3609-3611, which is incorporated in its entirety by reference, more preferably in the presence of diethyl azodicarboxylate (DEAD)/triphenylphosphine (TPP).

Preferably, said compound of formula (VIII), its enantiomers, or mixtures thereof, is obtained by oxidizing, preferably in the presence of IBX, a compound of formula (IX), its enantiomers, or mixtures thereof 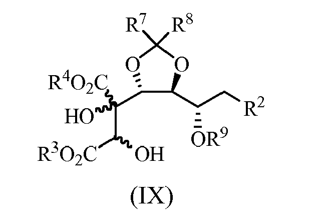 wherein
R², R³, R⁴, R⁵, R⁷, R⁸ and R⁹ are as defined above.

The oxidation with IBX, is preferably performed in the presence of ethyl acetate, which allows directly subjecting the compounds of formula (VIII) obtained to the following reaction step, without needing to purify them. Therefore, according to a preferred embodiment, the transformation of a compound of formula (IX), its enantiomers, or mixtures thereof, into a compound of formula (VII), its stereoisomers, especially its enantiomers, or mixtures thereof, is performed without isolating said compound of formula (VIII).

Therefore, another aspect of the present invention is aimed at compounds of formula (VIII) or (IX), their enantiomers, or mixtures thereof as defined above, which are intermediates which allow obtaining zaragozic acid and its derivatives of formula (XXVI).

According to a preferred embodiment, the compound of formula (IX), its enantiomers or mixtures thereof, is a compound of formula (IXa) or (IXb), its enantiomers or mixtures thereof: 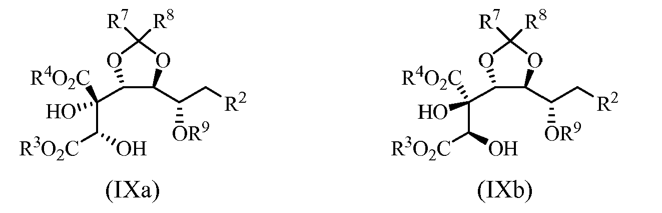 wherein
R², R³, R⁴, R⁵, R⁷, R⁸ and R⁹ are as defined above.

An additional aspect of the present invention is aimed at a process for the synthesis of a compound of formula (IX), its enantiomers, or mixtures thereof, **characterized in that** it comprises the dihydroxylation, preferably in the presence of OsO₄, of a compound of formula (X), its enantiomers, or mixtures thereof 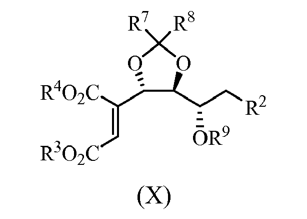 wherein
R², R³, R⁴, R⁷, R⁸ and R⁹ are as defined above.

The dihydroxylation of the compounds of formula (X) can proceed by attack on the alpha or beta side, giving rise to two possible compounds of formula (IX), their enantiomers or mixtures thereof, specifically compounds of formula (IXa) or (IXb), their enantiomers or mixtures thereof, mentioned above.

Both compounds are useful for the purposes of the present invention since, as has been seen above, it will then be possible to invert the configuration of carbon C7 in compounds of formula (VII) or (VII) by means of a Mitsunobu reaction.

Alternatively, said hydroxylation can be performed in an enantioselective manner. In this case, if the starting material is a racemate, one of the possible diastereoisomers would preferably be generated. It would therefore be a kinetic resolution of the starting racemate. See: Kolb, H. C.; Van Nievwenhze, M. S.; Sharpless, K. B. Chem. Rev. 1994, 94, 2483. Therefore, another aspect of the present invention is aimed at a compound of formula (X) its enantiomers, or mixtures thereof as defined above, which are intermediates which allow obtaining zaragozic acid and its derivatives of formula (XXVI).

An additional aspect of the present invention is aimed at a process for the synthesis of a compound of formula (X), its enantiomers, or mixtures thereof, **characterized in that** it comprises the acetalization or hemiacetalization of a compound of formula (XI), its enantiomers, or mixtures thereof 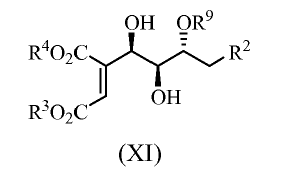 in the presence of a compound of formula (R₇)(R₈)C=O or hydrates or aryl or alkyl acetals or hemiacetals thereof
wherein

R², R³, R⁴, R⁷, R⁸ and R⁹ are as defined above.

The protection of the 1,2-diol group of a compound of formula (XI) as an acetal or hemiacetal can be performed by following methods known in the state of the art and allows maintaining this group stable throughout the synthesis. For conditions for the protection of 1,2-diols useful in the present invention see a) Konno, H.; Makabe, H.; Tanaka, A.; Oritani, T. Tetrahedron 1996, 52, 9399-9408; or b) Greene, T. W.; Wuts, P. G. M. Greene's Protective Groups in Organic Synthesis; John Wiley & Sons: Hoboken, 2007. pp.: 306-321, which are incorporated by reference. According to a preferred embodiment, R₇ and R₈ are methyl, or hydrogen or phenyl; or together with the carbon to which they are attached form a cyclohexane or cyclopentane ring. According to another preferred embodiment, R₇ is methyl and R₈ is phenyl. The compounds of formula (R₇)(R₈)C=O can be used in ketone form or in acetal or hemiacetal form. According to another preferred embodiment, a compound of formula (XI), its enantiomers, or mixtures thereof, is reacted with 2,2-dimethoxypropane in the presence of catalytic amounts of acid, preferably para-toluenesulfonic acid.

Therefore, another aspect of the present invention is aimed at a compound of formula (XI) its enantiomers, or mixtures thereof as defined above, which are intermediates which allow obtaining zaragozic acid and its derivatives of formula (XXVI).

An additional aspect of the present invention is aimed at a process for the synthesis of a compound of formula (XI), its enantiomers, or mixtures thereof, **characterized in that** it comprises the following steps
(i) reacting a compound of formula (XXI) in the presence of a compound of formula PY₃, and subsequently adding a compound of formula (XVI), its stereoisomers or mixtures thereof, to yield a compound of formula (XV), its stereoisomers or mixtures thereof 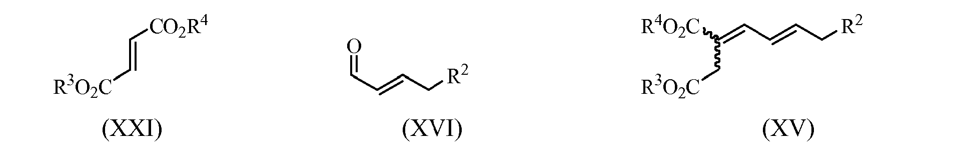
(ii) epoxidizing, preferably with meta-chloroperbenzoic acid (m-CPBA), said compound of formula (XV), to obtain a compound of formula (XIV), its stereoisomers, especially its enantiomers, or mixtures thereof, 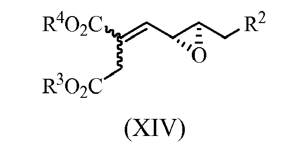
(iii) isomerizing in the presence of a base, preferably DBU, said compound of formula (XIV), to obtain a compound of formula (XIII), its stereoisomers, especially its enantiomers, or mixtures thereof, 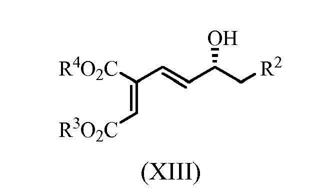
(iv) introducing a trialkylsilyl group into said compound of formula (XIII) to obtain a compound of formula (XII), its stereoisomers, especially its enantiomers, or mixtures thereof 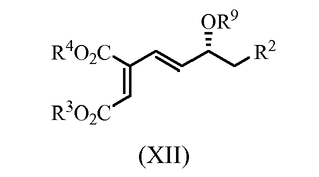 and
(v) dihydroxylating said compound of formula (XII) wherein
   R², R³, R⁴ and R⁹ are as defined above; and
   each of the Y groups is independently selected from among C₆-C₁₀ aryl groups or C₁-C₆ alkyl groups.

The preparation of compounds of formula (XV) (step (i)) can be performed according to processes described in the state of the art, and it has been performed at a multigram scale (Maryanoff, B. E.; Reitz, A. B. Chem. Rev. 1989, 89, 863-927; Trost, B. M.; Melvin, L. S. Jr. J. Am. Chem. Soc. 1976, 98, 1204-1212.). According to a preferred embodiment, the compound of formula PY₃ is n-Bu₃P. The compounds (XXI) and (XVI) can be commercially purchased or repaired according to established processes. The compounds of formula (XXI) are fumarate esters, preferably dimethyl fumarate. It is possible to purchase different fumarates, for example dimethyl or diisobutyl fumarates, among others.

Various compounds of formula (XVI) are also easily obtainable. Other compounds of formula (XVI) not commercially obtainable can be prepared according to methods similar to those described in, for example, a) Evans, D. A.; Barrow, J. C.; Leighton, J. L.; Robichaud, A. J.; Sefkow, M. J. Am. Chem. Soc. 1994, 116, 12111-12112 (compound **4**); b) Tomooka, K.; Kikuchi, M.; Igawa, K.; Suzuki, M.; Keong, P. - H.; Nakai, T. Angew. Chem. Int. Ed. 2000, 39, 4502-4505 (compounds **14, 15** or **16**); b) Nicolaou, K. C.; Yue, E. W.; Naniwa, Y.; De Riccardis, F.; Nadin, A.; Leresche, J. E.; La Greca, S.; Yang, Z. Angew. Chem. Int. Ed. Engl. 1994, 33, 2184-2187 (compound **6**); or c) Armstrong, A.; Jones, L. H.; Barsanti, P. A. Tetrahedron Lett. 1998, 39, 3337-3340 (compound **6**); or d) Evans, et al Tetrahedron Lett. 1993, 34, 8403 (see reference 4b in Carreira, E. M.; Du Bois, J. J. Am. Chem. Soc. 1994, 116, 10825-10826 for the preparation of compound **9**), all incorporated herein by reference.

The epoxidation of the compounds of formula (XV) (step (ii)) can be performed in the presence of epoxidizing agents such as meta-chloroperbenzoic acid, resulting in a compound of formula (XIV) in racemic form. Alternatively, the epoxidation can be performed by means of chiral reagents, giving rise to enantiomerically pure or enantiomerically enriched compounds of formula (XIV), which gives rise to the subsequent intermediates of formula (XIII) to (I) and (XXV) and (XXVI) (defined below) also being obtained in an enantiomerically pure or enantiomerically enriched manner. Therefore, the use at this point of a chiral epoxidizing agent allows obtaining zaragozic acid and its derivatives of formula (XXVI) in an enantiomerically pure or enantiomerically enriched manner. Some conditions useful for performing the epoxidation enantiomerically can be found in Jacobsen-Katsuki (see: Katsuki, T. Adv. Synth. Catal. 2002, 344, 131-147)2); or Shi (see: Wang, Z. X.; Tu, Y.; Frohn, M.; Zhang, J. R; Shi, Y. J. Am. Chem. Soc. 1997, 119, 11224-11235).

The isomerization of the compounds of formula (XIV) (step (iii)) allows opening the epoxide and isomerizing the double bond to provide a compound of formula (XIII). The base used is preferably DBU (1,8-diazabicyclo[5.4.0]undec-7-ene).

Non-illustrative examples of conditions in which the hydroxyl group of a compound of formula (XIII) (step (iv)) can be protected to obtain a compound of formula (XII) can be found in, for example, Dalla, V.; Catteau, J. P. Tetrahedron 1999, 55, 6497-6510, and the trialkylsilyl groups which can be used in this reaction, as well as reagents suitable for their introduction and removal, are known for the person skilled in the art (for example, see Greene, T. W.; Wuts, P. G. M. Greene's Protective Groups in Organic Synthesis; John Wiley & Sons: Hoboken, 2007).

According to a particular embodiment, the base used is imidazole and the silylating agent is TBDMSCl (tert-butyldimethylsilyl chloride). According to another preferred embodiment, the silylating agent is TBDMSOTf (tert-butyldimethylsilyl trifluoromethanesulfonate).

As has been mentioned above, the dihydroxylation (step (v)) can be performed under conditions known by the skilled person, as described in Smith, M. B.; March, J. March's Advanced Organic Chemistry; John Wiley & Sons: New York, 2001. pp.: 1048-1051. According to a preferred embodiment, the dihydroxylation is performed in the presence of osmium tetroxide/*N*-methylmorpholine-*N*-oxide or potassium permanganate.

Therefore, additional aspects of the present invention are aimed at compounds of formula (XII), (XIII), (XIV) or (XV), their enantiomers, or mixtures thereof as defined above, which are intermediates which allow obtaining zaragozic acid and its derivatives of formula (XXVI).

According to an additional aspect, the present invention relates to a process for preparing zaragozic acid and derivatives thereof of formula (XXVI), their stereoisomers, especially enantiomers, or mixtures thereof, **characterized in that** it comprises the following steps
(i) reacting in acidic medium a compound of formula (II), its enantiomers or mixtures thereof, or a compound of formula (III), its enantiomers or mixtures thereof, or a mixture of compounds of formula (II) and (III), as defined above; to obtain a compound of formula (I), its enantiomers or mixtures thereof, as defined above;
(ii) hydrolyzing in basic medium the ester groups of said compound of formula (I), its enantiomers or mixtures thereof, to provide a compound of formula (XXV), its enantiomers or mixtures thereof 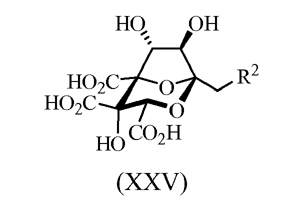 wherein
   R² is as defined above;
   and
(iii) reacting said compound of formula (XXV), its enantiomers or mixtures thereof, with a compound of formula (XXII) in the presence of a base, to obtain a compound of formula (XXVI), its enantiomers or mixtures thereof 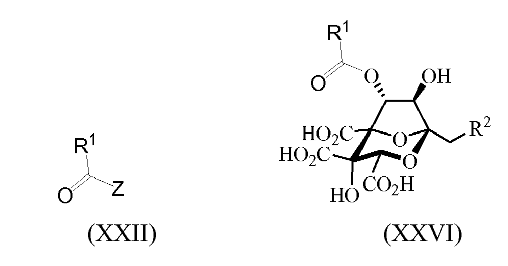
wherein
R¹ is selected from the group consisting of C₁-C₂₀ alkyl or C₁-C₂₀ alkenyl, which are unsubstituted or substituted with at least one group which is selected from the group consisting of C₁-C₄ alkyl; and/or a group in the end position which is selected from the group consisting of C₆-C₁₀ aryl;
Z is selected from the group consisting of hydroxyl and alkoxyl; and
R² is as defined above.

The hydrolysis of ester groups to provide the corresponding acid groups is known by the person skilled in the art (see, for example, Kocienski, P.J. Protecting Groups; Thieme: Stuttgart, 2000. pp.: 393-425). Non-limiting conditions for the purposes of the present invention are, preferably, those in which the hydrolysis of the compounds of formula (XXV) is performed in the presence of an alkali or alkaline earth metal hydroxide, for example in the presence of LiOH, NaOH, Ba(OH)₂, or in the presence of Na₂S.

The coupling of the compound of formula (XXII) involves the esterification of the compound of formula (XXV) to provide the compound of formula (XXVI). Suitable conditions for this transformation are known in the state of the art. For example, it can be performed according to the conditions described in Carreira, E. M.; Du Bois, J. J. Am. Chem. Soc. 1994, 116, 10825-10826, wherein the compound of formula (XXII) is an acid chloride (Z=Cl), described in Stoermer, D.; Caron, S.; Heathcock, C. H. J. Org. Chem. 1996, 61, 9115-9125, or in Evans, D. A.; Barrow, J. C.; Leighton, J. L.; Robichaud, A. J.; Sefkow, M. J. Am. Chem. Soc. 1994, 116, 12111-12112, wherein the compound of formula (XXII) is an acid (Z=OH) and the reaction proceeds in the presence of DMAP.

As can be seen, the sequence described in the present invention allows obtaining zaragozic acid and derivatives thereof of formula (XXVI), in a few steps, using reagents which are usual in the synthesis of organic compounds. Said synthesis can be performed by carrying from the start (compounds of formula (XVI)) the complete chain R². Alternatively, it is possible to start the synthesis with a compound of formula (XVI) which does not comprise a complete chain, but rather a precursor moiety thereof. The sequence can be performed as has been described above, and the complete chain can be constructed at the most convenient time, either by means of a single synthetic step or by means of consecutive or non-consecutive successive steps.

Therefore, according to a preferred embodiment R² is a C₁-C₅ alkyl or alkenyl substituted, preferably in the end position, with a hydroxyl group or a protected hydroxyl. At the time of constructing the chain, it will be possible to functionalize said hydroxyl such that it is activated for an alkylation reaction. For example, it is possible to oxidize the alcohol to aldehyde (for example, in the presence of PCC) and then react the resulting compound in the presence of an ylide (or vice versa) or then react with an alkynyl in the presence of a base. For example, see the formation of the compound 30 in Nakamura, S.; Hirata, Y.; Kurosaki, T.; Anada, M.; Kataoka, O.; Kitagaki, S.; Hashimoto, S. Angew. Chem. Int. Ed. 2003, 42, 5351-5355; or the formation of the compounds **57** and **58** in Kataoka, O.; Kitagaki, S.; Watanabe, N.; Kobayashi, J.; Nakamura, S.; Shiro, M.; Hashimoto, S. Tetrahedron Lett. 1998, 39, 2371-2374.

For example, different compounds of formula (XVI) can be prepared, wherein R² is a C₁-C₅ alkyl or alkenyl substituted in the end position with a hydroxyl group or a protected hydroxyl (alpha,beta-unsaturated aldehydes) from the corresponding hydroxyaldehyde and a suitable ylide (see Scheme 3 - R=hydrogen or protecting group). 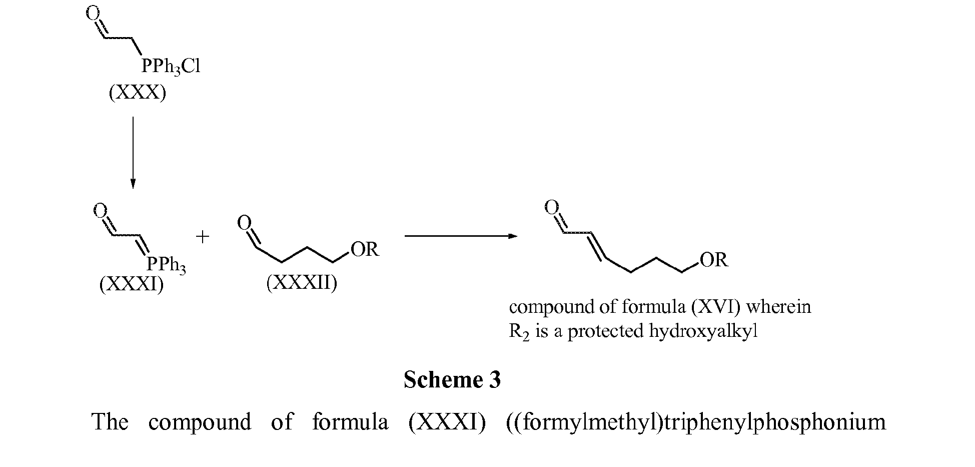 chloride) can be obtained, for example, according to methods described in Phytochemistry 1995, 38, 1169-1173, which is incorporated in its entirety by reference. The conditions for reacting a compound of formula (XXXI) and a compound of formula (XXXII) can be found in Tetrahedron 1993, 49, 10643-10654. Said document also teaches how to prepare a compound of formula (XXXII) from a diol (1,4-butanediol).

As has been indicated above, the compound of formula (XVI) can be constructed from the start with all the functionalities of the final compound of formula (XXVI). For example, reacting the compound of formula (XXX) with the compound 32 described in Carreira, et al., J. Am. Chem. Soc. 1995, 117, 8106-8125 following a process similar to the one shown in Scheme 3 would provide the compound of formula (XVI) necessary for obtaining, for example, zaragozic acid C (see Scheme 4).

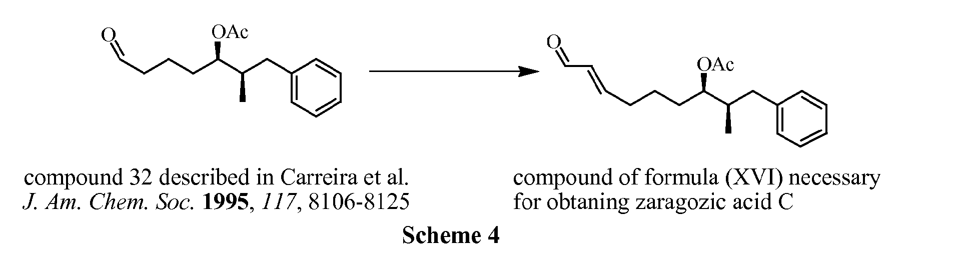

### Definitions

For the purpose of facilitating the understanding of the present invention, the meanings of several terms and expressions as they are used in the context of the invention are included herein.

"Alkyl" refers to a radical with a linear or branched hydrocarbon chain which consists of carbon and hydrogen atoms, which does not contain unsaturations and which is attached to the rest of the molecule by means of a single bond, for example, methyl, ethyl, propyl, isopropyl or n-butyl.

"Alkenyl" refers to a radical with a linear or branched hydrocarbon chain which consists of carbon and hydrogen atoms, which contains at least one unsaturation, and which is attached to the rest of the molecule by means of a single bond, for example, ethenyl, n-propenyl, i-propenyl, n-butenyl, n-pentenyl, etc.

"Alkylidene" refers to a radical with a linear hydrocarbon chain which consists of carbon and hydrogen atoms, and which is attached to the rest of the molecule from the two ends by means of single bonds to the same carbon atom, and therefore form a cycle, for example, ethylene (-CH₂-CH₂-), n-propylene (-CH₂-CH₂-CH₂-), n-butylene (-CH₂-CH₂-CH₂-CH₂-), n-pentylene (-CH₂-CH₂-CH₂-CH₂-CH₂-), etc. In the event of being a methylene group, it refers to the =CH₂ group.

"Halide" or "halogen" means-F, -Cl, -Br or -I;

A "stereoisomer" in the present application refers to compounds formed by the same atoms attached by the same sequence of bonds but having different three-dimensional structures which are not interchangeable.

"Enantiomer" is understood as the mirror image of a stereoisomerically pure compound. For the purposes of the invention, an enantiomer can be considered as a mixture of two enantiomers having an enantiomeric excess greater than 95%, preferably greater than 98%, more preferably greater than 99%, more preferably greater than 99,5%.

"Heteroaryl" preferably means a fraction of monocyclic or bicyclic hydrocarbon comprising 1 or 2 aromatic nuclei, said nuclei being attached with, and/or covalently linked to one another, al least one of such nuclei containing 1, 2, 3 or 4 heteroatoms independently selected from the group consisting of N, O and S, such as -pirrolyl, - furyl, -thienyl, -pyridyl, -quinolyl, -isoquinolyl, -indolyl, -oxazolyl, -isoxazolyl, -diazinyl, and the like.

"Aryl" refers to an aromatic hydrocarbon radical such as phenyl, or naphthyl.

"Arylalkyl" refers to an aryl group attached to the rest of the molecule through an alkyl group, for example, benzyl ("-(CH₂)-phenyl" or "Bn").

"Alkoxyl" refers to a radical of formula -O-R¹⁰, wherein R¹⁰ represents a group selected from the group consisting of substituted or unsubstituted alkyl, substituted or unsubstituted alkenyl, substituted or unsubstituted aryl, and substituted or unsubstituted arylalkyl.

"Alkylcaboxyhydroxyl" refers to a radical of formula R¹¹(C=O)O-, wherein R¹¹ is selected from the group consisting of C₁-C₆ alkyl, C₁-C₆ alkenyl, C₆-C₁₀ aryl and C₇-C₁₅ arylalkyl.

When the present application refers to a "protected hydroxyl", it indicates a hydroxyl group blocked such that it is inert to determined reactions and which can later be removed under controlled conditions. Said groups are known by the person skilled in the art and the most suitable ones can be selected according to the reactions to which the hydroxyl group is to be inert and/or the conditions under which said protecting group is to be removed, i.e., the conditions under which the hydroxyl group is to be taken off. Examples of suitable hydroxyl protecting groups and the conditions for their removal can be found in reference texts such as for example, Greene and Wuts' "Protective Groups in Organic Synthesis", John Wiley & Sons, Inc., New York, 4th Ed., 2007.

Preferred protecting groups for the purposes of the present invention are:
- silyl derivatives of formula -Si(R¹²)3 (commonly referred to as trialkylsilyls), such as trimethylsilyl ("TMS"), triethylsilyl, tert-butyldimethylsilyl ("TBDMS"), tert-butyldiphenylsilyl, tri-isopropylsilyl, diethylisopropylsilyl, texyldimethylsilyl ether, triphenylsilyl, di-tert-butylmethylsilyl;
- ethers of formula -R¹², such as methyl ether, tert-butyl ether, benzyl ether, p-methoxybenzyl ether , 3,4-dimethoxybenzyl ether, trityl ether; allyl ether;
- alkoxymethyl ethers of formula -CH₂-O-R¹², such as methoxymethyl ether, 2-methoxyethoxymethyl ether, benzyloxymethyl ether, p-methoxybenzyloxymethyl ether, 2-(trimethylsilyl)ethoxymethyl ether. The oxygen atom can be replaced by a sulfur atom to form an alkylthiomethyl ether of formula -CH₂-S-R¹², such as methylthiomethyl ether. Tetrahydropyranyl ethers and derivatives are also commonly used hydroxyl protecting groups;
- esters of formula -C(=O)R¹², acetate, benzoate; pivalate; methoxiacetate; chloroacetate; levulinate;
- carbonates of formula -C(=O)-O-R^{12,} such as benzyl carbonate, p-nitrobenzyl carbonate, tert-butyl carbonate, 2,2,2-trichloroethyl carbonate, 2-(trimethylsilyl)ethyl carbonate, or allyl carbonate; or
- sulfates of formula SO₃-O-R¹² or salts thereof, such as SO₃·pyridine.

In all the previous formulas, R¹² represents a group selected from the group consisting of substituted or unsubstituted C₁-C₁₂ alkyl, substituted or unsubstituted C₁-C₁₂ alkenyl, substituted or unsubstituted C₆-C₁₀ aryl, and substituted or unsubstituted C₇-C₁₅ arylalkyl.

The references of the present document to substituted groups in the compounds of the present invention refer to the specified moiety which can be substituted in one, two or three available positions with one, two, three suitable groups, which are independently selected from the group consisting of cyano; alkanoyl, such as a C₁-C₆ alkanoyl group, such as acyl and the like; carboxamido (-(C=O)NH₂); trialkylsilyl; carbocyclic aryl having 6 or more carbons, particularly phenyl or naphthyl and (C₁-C₃)alkylaryl such as tolyl. As a non-limiting example, "substituted alkyl" includes groups such as cyanoethyl, acetylmethyl, carboxamidomethyl (-CH₂CONH₂), 2-trimethylsilylethyl, benzyl, diphenylmethyl.

In each case, when the number of carbon atoms of the corresponding "Cx-Cy" group is specified, it indicates that the group comprises between "x" and "y" carbon atoms. For example, when "C₁-C₃ alkyl" is indicated, it refers to an alkyl group of one, two or three carbon atoms, i.e., methyl, ethyl, propyl, or isopropyl. For example, when "C₁₀-C₁₅ alkyl" is indicated, it refers to an alkyl group of ten, eleven, twelve, thirteen, fourteen or fifteen carbon atoms, such as decyl, undecyl, dodecyl, tridecyl, tetradecyl or pentadecyl.

Unless otherwise indicated, the compounds of the invention also refer to those including compounds which differ only in the presence of one or more isotopically enriched atoms. For example, the compounds having the present structures, with the exception of the substitution of a hydrogen with a deuterium or with tritium, or the substitution of a carbon with a ¹³C- or ¹⁴C-enriched carbon, are within the scope of this invention.

The following examples illustrate different embodiments of the invention and must not be considered as limiting the scope thereof.

### Examples

### General methods and materials

All the reactions were performed under an argon atmosphere, except those indicated in each case. The solvents used were distilled and dried under an argon atmosphere. The reagents and solvents used are from the companies Aldrich, Fluka, Merck, Sigma, Acros, Lancaster, SDS or Scharlau, and were purified by usual processes when necessary. The purification of the reaction products was performed by column chromatography under pressure (flash chromatography), using 60 Merck silica gel (with a 230-400 mesh particle size) as a stationary phase.

The (fully decoupled) ¹H and ¹³C nuclear magnetic resonance spectra were performed at room temperature in the solvent indicated in each case (CDCl₃ and CD₃OD) using the following apparatuses: Varian Gemini-200 (200 MHz), Varian INOVA-300 (300 MHz), Bruker Avance-300 (300 MHz) and Varian INOVA-400 (400 MHz). The values of the chemical shifts are expressed in parts per million (δ, ppm), using as an internal reference the residual signal of the solvent: CDCl₃, 7.26 ppm (¹H-NMR) and 77.0 ppm (¹³C-NMR); CD₃OD, 3.31 ppm (¹H-NMR) and 49.0 ppm (¹³C-NMR). The ¹H-NMR spectra are described indicating the number of protons and the apparent multiplicity of each signal. The coupling constants (J) are the apparent ones and are expressed in Hz. The following abbreviations have been used: s (singlet), d (doublet), t (triplet), c (quadruplet), q (quintuplet) and m (multiplet).

The melting points (m.p.) were measured in a Reichert brand Kofler microscope. The infrared (IR) spectra were recorded in the Perkin-Elmer spectrophotometer models 681 and FT-IR Spectrum One. The low resolution mass spectra (LRMS) were recorded: (1) by direct injection of the sample into a Hewlett Packard 5973 MSD spectrophotometer using the electron impact (EI) ionization technique; or (2) in a Hewlett Packard LCMS 1100 MSD spectrophotometer (an HPLC-coupled quadrupole analyzer) using the electrospray chemical ionization technique (API-ES) in positive or negative modes. The elemental analyses (E.A.) were performed with the Perkin-Elmer 240C and Heraus CHN-O-Rapid analyzers.

Unless otherwise indicated, all the products shown in the examples are racemic (*rac*).

### Example 1: Preparation of methyl (3E,5E)-3-(methoxycarbonyl)-3,5-tridecadienoate (58)

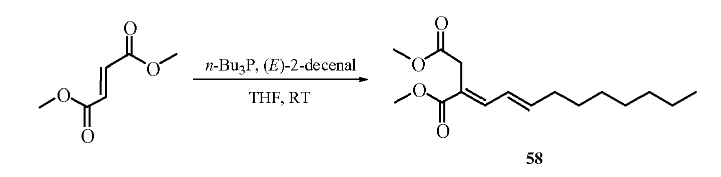

*n*-Bu₃P (7.12 g, 35.2 mmoles) was added to a solution of dimethyl fumarate (3.97 g, 27.6 mmoles) and (E)-2-decenal (3.89 g, 25.2 mmoles) in THF (44 ml). The mixture was stirred at room temperature for 48 hours. After that time, AcOEt (30 ml) and H₂O (30 ml) were added. The phases were separated, and the aqueous phase was extracted with AcOEt (3 × 25 ml). The organic phase was dried with anhydrous Na₂SO₄, filtered and the solvent was removed under reduced pressure. The product was purified by a chromatographic column (hexane/AcOEt, 20:1), obtaining (5.03 g, yield 71%) methyl (3E,5E)-3-(methoxycarbonyl)-3,5-tridecadienoate (**58**), as a transparent oil.
**IR**(NaCl): ν 2949, 2928, 2856, 1744, 1714, 1641, 1436, 1324, 1258, 1198, 1170, 1085, 975 cm⁻¹.
**¹H-NMR** (200 MHz, CDCl₃). δ 7.33 (1H, d, *J* = 10.4 Hz, H-4), 6.22 (1H, dt, *J* = 7.0, 15.1 Hz, H-6), 6.20 (1H, dd, *J* = 10.4, 15.1 Hz, H-5), 3.75 (3H, s, -OCH₃), 3.68 (3H, s,-OCH₃), 3.43 (2H, s, H-2), 2.18 (2H, m, H-7), 1.40-1.20 (10H, m, -CH₂-), 0.87 (3H, m,-CH₃).
**¹³C-NMR** (50 MHz,CDCl₃). δ 171.0, 167.6, 145.8, 141.5, 124.9, 121.5, 51.7, 51.6, 33.1, 32.0, 31.5, 28.9, 28.8, 28.5, 22.4, 13.9.
**LRMS(EI):** *m*/*z* 282(M⁺, 28), 251(16), 222(12),190(8),183(100),166(12),137(43). **E.A.** (C₁₆H₂₆O₄): Found: C, 68.20, H, 9.30; Calculated: C, 68.06, H, 9.28.

### Example 2: Preparation of methyl rac-(E,5S,6S)-5,6-epoxy-3-(methoxycarbonyl)-3-tridecenoate (60)

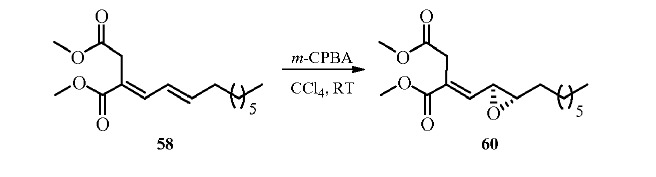

*m*-CPBA (3.66 g, 21.2 mmoles) was added to a solution of methyl (3E,5E)-3-(methoxycarbonyl)-3,5-tridecadienoate **(58)** (3.0 g, 10.62 mmoles) in CCl₄, (120 ml). The mixture was stirred at room temperature for 24 hours. After that time, the solvent was removed under reduced pressure. The crude reaction product was dissolved in AcOEt (50 ml) and washed with sat. NaHCO₃ (10 × 10 ml), dried with anhydrous Na₂SO₄, filtered and the solvent was removed under reduced pressure. The product was purified by a chromatographic column (hexane/AcOEt, 7:1), obtaining (1.93 g, yield 61%) methyl *rac*-(*E*,5*S*,6*S*)-5,6-epoxy-3-(methoxycarbonyl)-3-tridecenoate (**60**), as a transparent oil.
**IR** (NaCl): ν 3468, 2949, 2857, 1738, 1721, 1655, 1575, 1437, 1315, 1265, 1202, 1173, 1080, 1012, 932, 865, 776 cm⁻¹.
**¹H-NMR** (300 MHz, CDCl₃). δ 6.55 (1H, d, *J* = 8.3 Hz, H-4), 3.75 (3H, s, -OCH₃),3.70 (3H, s, -OCH₃), 3.57 (1H, d, *J* = 16.6 Hz, H-2), 3.44 (1H, d, *J* = 16.6 Hz, H-2), 3.28 (1H dd, *J* = 2.1, 8.3 Hz, H-5), 2.97 (1H, dt, *J* = 2.1, 5.3 Hz, H-6), 1.59 (2H, m, -CH₂-), 1.44 (2H, m, -CH₂-), 1.26 (8H, s_{broad}, -CH₂-), 0.87 (3H, t, *J* = 5.1 Hz, -CH₃).
**¹³C-NMR** (75 MHz, CDCl₃). δ 170.7, 166.4, 141.8, 131.2, 60.4, 54.3, 52.2, 52.2, 32.3, 31.8, 31.6, 29.2, 29.1, 25.7, 22.5, 14.0.
**LRMS(EI):** *m*/*z* 298(M⁺, 0), 282(0), 266(1), 237(2), 206(2), 179(3), 170(100), 139(10), 111(80).
**E.A.** (C₁₆H₂₆0₅): Found: C, 64.25, H, 8.95; Calculated: C, 64.41, H, 8.78.

### Example 3: Reaction of methyl rac-(E,5S,6S)-5,6-epoxy-3-(methoxycarbonyl)-3-tridecenoate (60) with DBU

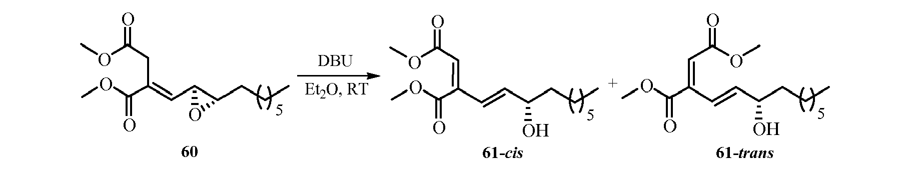

DBU (4.31 g, 28.35 mmoles) was added to a solution of methyl *rac*-(*E*,5*S*,6*S*)-5,6-epoxy-3-(methoxycarbonyl)-3-tridecenoate **(60)** (5.64 g, 18.90 mmoles) in Et₂O (190 ml). The mixture was stirred at room temperature for 20 minutes. After that time, Celite was added and the solvent was removed under reduced pressure. The product was purified by a chromatographic column (hexane/AcOEt, 4:1), obtaining (3.46 g, yield 61%) methyl *rac*-(2*Z*,4*E*,*S*)-6-hydroxy-3-(methoxycarbonyl)-2,4-tridecadienoate **(61-*cis*)** and methyl *rac*-(2*E*,4*E*,*S*)-6-hydroxy-3-(methoxycarbonyl)-2,4-tridecadienoate of **(61-*trans*)** in a 2:1 ratio, respectively, both as a colorless oil.

### Methyl rac-(2Z,4E,S)-6-hydroxy-3-(methoxycarbonyl)-2,4-tridecadienoate (61-cis)

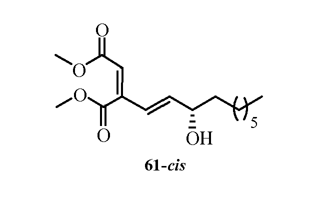

**IR**(NaCl): ν 3431, 2949, 2928, 2856, 1738, 1722, 1634, 1613, 1436, 1375, 1275, 1203, 1170, 1152, 1018, 968, 843 cm⁻¹.
**¹H-NMR** (300 MHz, CDCl₃). δ 6.32 (1H, d, *J* = 15.7 Hz, H-4), 5.98 (1H, dd, *J* = 5.3, 15.7 Hz, H-5), 5.84 (1H, s, H-2), 4.24 (1H, m, H-6), 3.89 (3H, s, -OCH₃), 3.72 (3H, s, - OCH₃), 1.84-1.52 (4H, m, -CH₂-), 1.25 (8H, s_{broad}, -CH₂-), 0.86 (3H, t, *J* = 6.5 Hz, - CH₃).
**¹³C-NMR** (75 MHz, CDCl₃). δ 167.7, 165.4, 147.2, 143.2, 125.1, 118.7, 71.7, 52.5, 51.8, 36.8, 31.7, 29.3, 29.1, 25.2, 22.5, 14.0.
**LRMS(EI)**: *m*/*z* 298(M⁺, 0), 280(0), 266(6), 234(24), 167(41), 153(10), 139(100), 127(58).
**E.A.** (C₁₆H₂₆O₅): Found: C, 64.30, H, 8.90; Calculated: C, 64.41, H, 8.78.

### Methyl rac-(2E,4E,S)-6-hydroxy-3-(methoxycarbonyl)-2,4-tridecadienoate (61-trans)

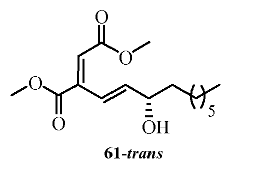

**IR** (NaCl): ν 3431,2928,2856,1723,1634,1600,1435,1206,1125, 1019, 980, 883 cm⁻¹.
**¹H-NMR** (300 MHz, CDCl₃). δ 7.38 (1H, d, *J* = 16.1 Hz, H-4), 6.46 (1H, dd, *J* = 6.1, 16.1 Hz, H-5), 6.33 (1H, s, H-2), 4.25 (1H, m, H-6), 3.82 (3H, s, -OCH₃), 3.75 (3H, s, - OCH₃),1.85-1.56 (4H, m, -CH₂-), 1.26 (8H, s_{broad}, -CH₂-), 0.86 (3H, m, -CH₃).
**¹³C-NMR** (75 MHz, CDCl₃). δ 166.7, 165.8, 144.3, 143.0, 122.5, 121.2,72.7,52.5, 51.7, 36.9, 31.7, 29.4, 29.1, 25.2, 22.6, 14.1.
**LRMS(EI)**: *m*/*z* 298(M⁺, 0), 281(0), 267(7), 234(18), 207(7), 179(4), 169(90), 139(100),127(41).
**E.A.** (C₁₆H₂₆O₅): Found: C, 64.32, H, 8.87; Calculated: C, 64.41, H, 8.78.

### Example 4: Preparation of methyl rac-(2Z,4E,S)-6-(tert-biqldimethylsilyloxy)-3-(methoxycarbonyl)-2.4-tridecadienoate (62-cis)

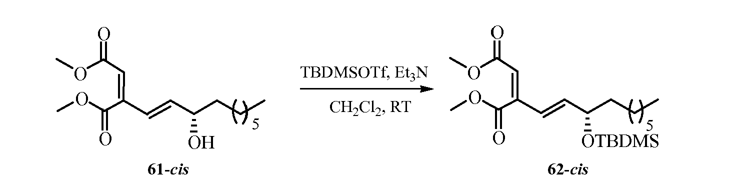

TBDMSOTf (0.403 g, 1.52 mmoles) was added to a solution of methyl *rac-*(2*Z*,4*E*,*S*)-6-hydroxy-3-(methoxycarbonyl)-2,4-tridecadienoate (**61-*cis*)** (0.380 g, 1.27 mmoles) and Et₃N (0.167 g, 1.65 mmoles) in CH₂Cl₂ (13.5 ml) at 0°C. The mixture was stirred at room temperature for 18 hours. After that time, AcOEt (10 ml) and Celite were added, and the solvent was removed under reduced pressure. The product was purified by a chromatographic column (hexane/AcOEt, 10:1), obtaining (0.430 g, yield 82%) methyl *rac*-(2*Z*,4*E*,*S*)-6-(*tert*-butyldimethylsilyloxy)-3-(methoxycarbonyl)-2,4-tridecadienoate (**62-*cis***), as a colorless oil.
**IR** (NaCl): ν 3426, 2949, 2929, 2857, 1744, 1723, 1634, 1614, 1461, 1438, 1375, 1271, 1253, 1202, 1169, 1152, 1094, 967, 837, 807, 777 cm⁻¹.
**¹H-NMR** (300 MHz, CDCl₃). δ 6.27 (1H, d, *J* = 15.8 Hz, H-4), 5.97 (1H, dd, *J* = 5.1, 15.8 Hz, H-5), 5.80 (1H, s, H-2), 4.22 (1H, m, H-6), 3.89 (3H, s, -OCH₃), 3.73 (3H, s, - OCH₃), 1.57 (2H, m, -CH₂-), 1.25 (10H, s_{broad}, -CH₂-), 0.89 (9H, s, *tert*-BuSi), 0.87 (3H, t, *J* = 6.5 Hz, -CH₃), 0.04 (3H, s, MeSi), 0.01 (3H, s, MeSi).
**¹³C-NMR** (75 MHz, CDCl₃). δ 167.8, 165.6, 147.6, 144.4, 124.5, 118.0, 72.2, 52.4, 51.8, 37.6, 31.7, 29.5, 29.1, 25.8, 25.3, 24.9, 22.6,18.2, 14.1, 14.0, -4.5, -4.8.
**LRMS(EI)**: *m*/*z* 412(M⁺, 0), 397(2), 380(5), 355(100), 323(58), 313(60),191(39). **E.A.** (C₂₂H₄₀O₅Si): Found: C, 64.10, H, 9.85; Calculated: C, 64.04, H, 9.77.

### Example 5: Reaction of methyl rac-(2Z,4E,S)-6-(tert-butyldimethylsilyloxy)-3-(methoxycarbonyl)-2,4-tridecadienoate (62-cis) with OsO₄/NMO

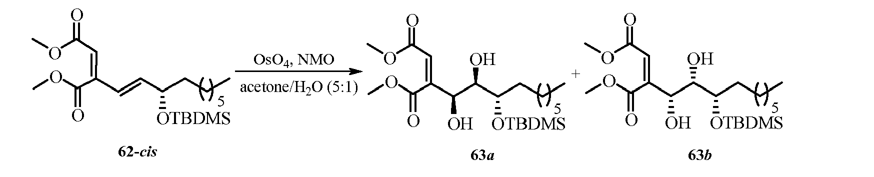

OsO₄ (2.5% in *tert-*BuOH*,* 0.015 g, 0.062 mmoles) was added to a solution of methyl *rac*-(2*Z*,4*E*,*S*)-6-(*tert*-butyldimethylsilyloxy)-3-(methoxycarbonyl)-2,4-tridecadienoate (**62-*cis***) (0.430 g, 1.042 mmoles) and NMO (0.134 g, 1.14 mmoles) in a 5:1 acetone/H₂O mixture (4.8 ml). The mixture was stirred at room temperature for 24 hours. After that time, AcOEt (10 ml) and Celite were added, and the solvent was removed under reduced pressure. The product was purified by a chromatographic column (hexane/AcOEt, 5:1), obtaining (0.270 g, yield 65%) a mixture in a 4:1 ratio of methyl *rac-*(*Z*,4*S*,5*R*,6*S*)-6-(*tert*-butyldimethylsilyloxy)-4,5-dihydroxy-3-(methoxycarbonyl)-2-tridecenoate (**63*a***) and methyl *rac*-(Z,4*R*,5*S*,6*S*)-6-(*tert-*butyldimethylsilyloxy)-4,5-dihydroxy-3-(methoxycarbonyl)-2-tridecenoate (**63*b***)*,* respectively, as a colorless oil.
**IR**(NaCl): ν 3463, 2949, 2929, 2857, 1730, 1654, 1436, 1360, 1258, 1201, 1170, 1089, 837, 776 cm⁻¹.
**¹H-NMR** (300 MHz, CDCl₃). δ 6.27 (1H, d, *J* = 1.9 Hz, H-2 **63*a***), 6.17 (1H, d, *J* = 1.4 Hz, H-2 **63*b***), 4.76 (1H, s_{broad}, H-4 **63*a***)*,* 4.35 (1H, ddd, *J* = 1.4,3.6,5.1 Hz, H-4 **63*b***)*,* 3.94 (1H, dt, *J* = 3.9, 6.6 Hz, H-6 **63*a***), 3.91 (1H, d, *J* = 2.9 Hz, -OH₄ **63*a***), 3.82 (3H, s, -OCH₃ **63*b***), 3.81 (3H, s, -OCH₃ **63*a***), 3.78 (1H, m, H-6 **63*b***), 3.73 (3H, s, -OCH₃ **63*a*, 63*b***), 3.64 (1H, m, H-5 **63*b***), 3.58 (1H, dd, *J* = 3.9, 7.8 Hz, H-5 **63*a***), 3.27 (1H, d, *J* = 4.8 Hz, -OH **63*b***), 2.71 (1H, d, *J* = 6.8 Hz, -OH **63*b***), 2.55 (1H, d, *J* = 7.8 Hz, -OH₅ **63*a***), 1.57 (2H, m, H-7 **63*a***, **63*b***), 1.26 (10H, s_{broad}, -CH₂-), 0.89 (9H, s, *tert*-BuSi), 0.88 (3H, m, -CH₃), 0.12 (3H, s, MeSi **63*a***), 0.11 (3H, s, MeSi **63*b***), 0.10 (3H, s, MeSi **63*a***), 0.09 (3H, s, MeSi **63*b***).
**¹³C-NMR** (75 MHz, CDCl₃). δ 167.7, 167.5, 165.7, 165.2, 148.6, 148.1, 121.9, 121.7, 76.0, 73.1, 72.2, 71.4, 70.4, 52.5, 52.5, 52.0, 51.9, 33.9, 31.7, 29.6, 29.1, 25.8, 25.7, 24.9, 22.5, 21.0, 17.9, 14.1, 14.0, -4.5, -4.7.

LRMS(EI): *m*/*z* 445(M⁺-1, 0), 415(3), 389(2), 357(16), 297(5), 243(92), 215(100), 142(34).

### Example 6: Preparation of methyl rac-(Z,4S,5R,6S)-6-(tert-butyldimethylsilyloxy)-4,5-(dimethylmethylenedioxy)-3-(methoxycarbonyl)-2-tridecenoate (64)

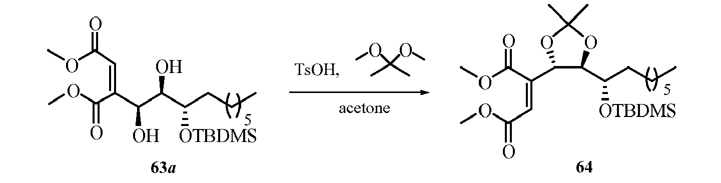

TsOH (0.020 g, cat.) was added to a solution of methyl *rac-*(*Z*,4*S*,5*R*,6*S*)-6-(*tert-*butyldimethylsilyloxy)-4,5-dihydroxy-3-(methoxycarbonyl)-2-tridecenoate (**63*a***) (1.36 g, 3.04 mmoles) and 2,2-dimethoxypropane (0.951 g, 9.13 mmoles) in acetone (6 ml) at 0°C. The mixture was stirred at room temperature for 5 hours. After that time, Et₂O(20 ml) was added, and the mixture was washed with sat. NaHCO₃ (2 × 10 ml), dried with anhydrous MgSO₄, filtered and the solvent was removed under reduced pressure. The product was purified by a chromatographic column (hexane/AcOEt, 5:1), obtaining (1.34 g, yield 91%) methyl *rac*-(*Z*,4*S*,5*R*,6*S*)-6-(*tert*-butyldimethylsilyloxy)-4,5-(dimethylmethylenedioxy)-3-(methoxycarbonyl)-2-tridecenoate (**64**), as a colorless oil.
**IR**(NaCl): ν 3444, 2985, 2949, 2930, 2857, 1734, 1655, 1461, 1436, 1381, 1256, 1167, 1068, 836, 776 cm⁻¹.
**¹H-NMR** (300 MHz, CDCl₃). δ 6.08 (1H, s, H-2), 4.68 (1H, d, *J* = 7.5 Hz, H-4), 4.23 (1H, dd, *J* = 3.1, 7.5 Hz, H-5), 3.86 (1H, m, H-6), 3.84 (3H, s, -OCH₃), 3.74 (3H, s, - OCH₃), 1.42 (3H, s, -CH₃), 1.32 (3H, s, -CH₃), 1.24 (12H, s_{broad}, -CH₂-), 0.89 (9H, s, *tert*-BuSi), 0.88 (3H, m, -CH₃), 0.07 (6H, s, Me₂Si).
**¹³C-NMR** (75 MHz, CDCl₃). δ 167.0, 164.7, 149.0, 121.3, 109.7, 81.3, 76.5, 71.5, 52.3, 52.0, 33.9, 31.7, 29.1, 29.1, 27.1, 26.3, 25.9, 25.1, 22.6, 18.1, 14.0, -4.4, -4.4.
**LRMS(EI)**: *m*/*z* 486(M⁺, 0), 471(4), 455(0), 429(44), 411(6), 397(1), 371(7), 339(34), 321(3), 311(7), 279(7), 243(100), 156(36).
**E.A.** (C₂₅H₄₆O₇Si): Found: C, 61.60, H, 9.60; Calculated: C, 61.69, H, 9.53.

### Example 7: Reaction of methyl rac-(Z,4S,5R,6S)-6-(tert-Butyldimethylsilyloxy)-4,5-(dimethylmethylenedioxy)-3-(methoxycarbonyl)-2-tridecenoate (64) with OsO₄

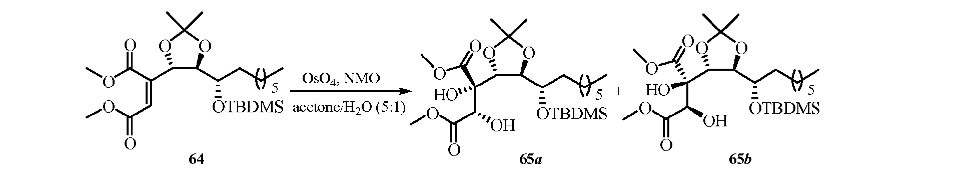

OsO₄ (2.5% in *tert-*BuOH*,* 0.030 g, 0.12 mmoles) was added to a solution of methyl *rac*-(*Z*,4*S*,5*R*,6*S*)-6-(*tert*-butyldimethylsilyloxy)-4,5-(dimethylmethylenedioxy)-3-(methoxycarbonyl)-2-tridecenoate (**64**) (0.980 g, 2.01 mmoles) and NMO (0.518 g, 4.43 mmoles) in a 5:1 acetone/H₂O mixture (9.2 ml). The mixture was stirred at room temperature for 5 days. After that time, an aqueous solution of 5% Na₂S₂O₃ (0.5 ml), AcOEt (20 ml) and Celite were added, and the solvent was removed under reduced pressure. The product was purified by a chromatographic column (hexane/ AcOEt, 10:1), obtaining (0.320 g, yield 31%) methyl *rac-*(2*S*,3*S*,4*R*,5*R*,6*S*)-6-(*tert-*butyldimethylsilyloxy)-2,3-dihydroxy-4,5-(dimethylmethylenedioxy)-3-(methoxycarbonyl)tridecanoate (**65*a***) and (0.322 g, yield 31%) methyl *rac-*(2*R*,3*R*,4*R*,5*R*,6*S*)-6-(*tert*-butyldimethylsilyloxy)-2,3-dihydroxy-4,5-(dimethylmethylenedioxy)-3-(methoxycarbonyl)tridecanoate (65b), both as a colorless oil.

### Methyl rac-(2S,3S,4R,5R,6S)-6-(tert-butyldimethylsilyloxy)-2,3-dihydroxy-4,5-(dimethylmethylenedioxy)-3-(methoxycarbonyl)tridecanoate (65a)

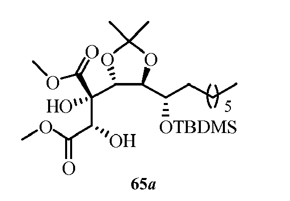

**IR**(NaCl): ν 3490, 2949, 2930, 2857, 1748, 1461, 1439, 1379, 1367, 1255, 1213, 1109, 1088, 1000, 837, 775 cm⁻¹.
**¹H-NMR** (300 MHz, CDCl₃). δ 4.75 (1H, d, *J* = 5.8 Hz, H-4), 4.43 (1H, s, H-2), 4.41 (1H, dd, *J* = 5.8, 9.7 Hz, H-5), 3.85 (3H, s, -OCH₃), 3.80 (1H, m, H-6), 3.76 (3H, s, - OCH₃),1.73 (1H, m), 1.59 (1H, m), 1.36 (3H, s, -CH₃), 1.35 (3H, s, -CH₃), 1.26 (10H, s_{broad}, -CH₂-), 0.92 (9H, s, *tert*-BuSi), 0.91 (3H, m, -CH₃), 0.16 (3H, s, MeSi), 0.14 (3H, s, MeSi).
**¹³C-NMR** (75 MHz, CDCl₃). δ 172.0, 170.7, 110.1, 80.8, 79.6, 78.0, 74.7, 73.2, 52.8, 52.5, 33.3, 31.6, 29.6, 29.0, 27.7, 27.0, 26.9, 25.7, 22.8, 22.4, 18.0, 13.8, 0.8, -4.3, -4.6. **LRMS(EI)**: *m*/*z* 520(M⁺, 0), 463(0), 431(1), 405(4), 369(13), 327(5), 295(5), 243(100), 187(18), 73(60).
**E.A.** (C₂₅H₄₈O₉Si): Found: C, 57.60, H, 9.35; Calculated: C, 57.66, H, 9.29.

### Methyl rac-(2R,3R,4R,5R,6S)-6-(tert-butyldimethylsilyloxy)-2,3-dihydroxy-4,5-(dimethylmethylenedioxy)-3-(methoxycarbonyl)tridecanoate (65b)

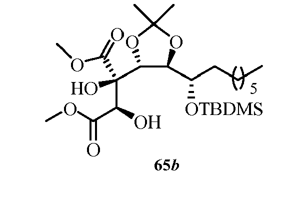

**IR** (NaCl): v 3489, 2949, 2929, 2857, 1748, 1461, 1438, 1380, 1367, 1252, 1216, 1101, 1050, 1003, 836, 775 cm⁻¹.
**¹H-NMR** (300 MHz, CDCl₃). δ 4.65 (1H, m, H-4), 4.36 (1H, s, H-2), 4.34 (1H, m, H-5), 3.80 (3H, s, -OCH₃), 3.79 (3H, s, -OCH₃), 3.76 (1H, m, H-6), 1.61 (2H, m), 1.41 (3H, s, -CH₃), 1.39 (3H, s, -CH₃), 1.26 (10H, s_{broad}, -CH₂-), 0.90 (9H, s, *tert*-BuSi), 0.89 (3H, m, -CH₃), 0.09 (3H, s, MeSi), 0.08 (3H, s, MeSi).
**¹³C-NMR** (75 MHz, CDCl₃). δ 171.1, 170.9, 110.2, 78.9, 78.8, 76.9, 72.7, 71.1, 52.8, 31.6, 29.4, 29.0, 27.6, 26.5, 25.7, 24.8, 22.4, 18.0, 13.8, -4.4, -4.6.
**LRMS(EI):** *m*/*z* 520(M⁺, 0), 505(2), 463(0), 431(1), 387(9), 327(4), 299(6), 243(100), 187(19), 73(69).
**E.A.** (C₂₅H₄₈O₉Si): Found: C, 57.58, H, 9.37; Calculated: C, 57.66, H, 9.29.

### Example 8: Preparation of methyl rac-(Z,4S,5R,6R,7S)-7-(tert-butyldimethylsilyloxy)-5,6-(dimethylmethylenedioxy)-4-hydroxy-3,4-bis(methoxycarbonyl)-2-tetradecenoate (76a)

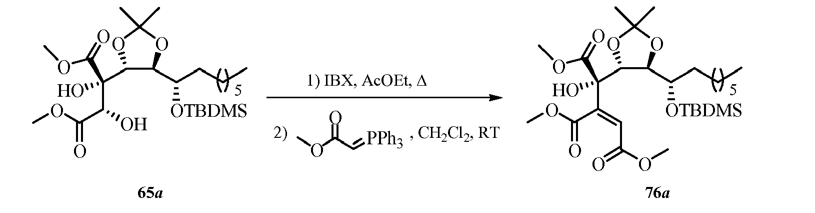

IBX (0.474 g, 1.69 mmoles) was added to a solution of methyl *rac-*(2*S*,3*S*,4*R*,5*R*,6*S*)-6-(*tert*-butyldimethylsilyloxy)-2,3-dihydroxy-4,5-(dimethylmethylenedioxy)-3-(methoxycarbonyl)tridecanoate (**65*a***) (0.294 g, 0.564 mmoles) in AcOEt (5 ml). The mixture was heated at 80°C for 8 hours. After that time, the mixture was filtered under vacuum over Celite and the solvent was removed under reduced pressure. The residue was dissolved in CH₂Cl₂ (10 ml), and [(methoxycarbonyl)methylene]triphenylphosphorane (0.451 g, 1.34 mmoles) was added. The mixture was stirred at room temperature for 24 hours. After that time AcOEt (10 ml) and Celite were added, and the solvent was removed under reduced pressure. The product was purified by a chromatographic column (hexane/AcOEt, 5:1), obtaining methyl *rac*-(*Z*,4*S*,5*R*,6*R*,7*S*)-7-(*tert*-butyldimethylsilyloxy)-5,6-(dimethylmethylenedioxy)-4-hydroxy-3,4-bis(methoxycarbonyl)-2-tetradecenoate (**76*a***) (0.220 g, yield 71%), as a colorless oil.
**IR** (NaCl): v 3477, 2949, 2929, 2854, 1743, 1642, 1461, 1435, 1367, 1252, 1213, 1166, 1095, 1062, 836, 774 cm⁻¹.
**¹H-NMR** (400 MHz, CDCl₃). δ 6.49 (1H, s, H-2), 4.45 (1H, d, *J* = 7.3 Hz, H-5), 4.28 (1H, dd, *J* = 2.6, 7.3 Hz, H-6), 4.00 (1H, s, -OH), 3.85 (3H, s, -OCH₃), 3.81 (3H, s, - OCH₃), 3.77 (1H, m, H-7), 3.73 (3H, s, -OCH₃), 1.63-1.42 (2H, m, H-8), 1.38 (6H, s, - CH₃), 1.28 (10H, s_{broad}, -CH₂-), 0.89 (3H, m, -CH₃), 0.88 (9H, s, *tert*-BuSi), 0.02 (3H, s, MeSi), 0.02 (3H, s, MeSi).
**¹³C-NMR** (100 MHz, CDCl₃). δ 171.0, 166.3, 164.5, 147.1, 122.5, 110.1, 79.9, 79.8, 78.4, 72.7, 53.9, 52.5, 52.0, 31.8, 31.2, 29.8, 29.3, 27.7, 26.3, 25.9, 25.7, 25.4, 22.6, 18.2, 14.0, -4.4.
**LRMS(EI):** *m*/*z* 574(M⁺, 0), 517(3), 485(2), 409(5), 335(14), 257(13), 243(100), 215(31), 171(16).
**E.A.** (C₂₈H₅₀O₁₀Si): Found: C, 58.60, H, 8.85; Calculated: C, 58.51, H, 8.77.

### Example 9: Preparation of methyl rac-(Z,4R,5R,6R,7S)-7-(tert-butyldimethylsilyloxy)-5,6-(dimethylmethylenedioxy)-4-hydroxy-3,4-bis(methoxycarbonyl)-2-tetradecenoate

(76*b*)

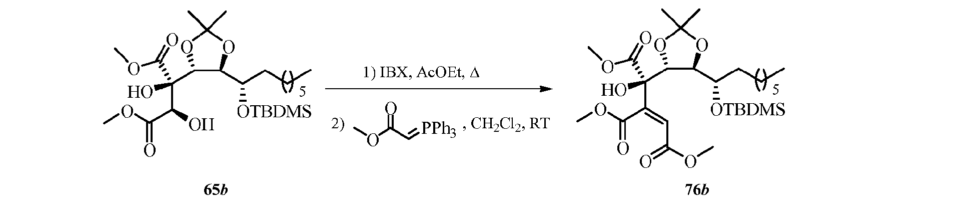

IBX (0.519 g, 1.85 mmoles) was added to a solution of methyl *rac-*(2*R*,3*R*,4*R*,5*R*,6*S*)-6-(*tert*-butyldimethylsilyloxy)-2,3-dihydroxy-4,5-(dimethylmethylenedioxy)-3-(methoxycarbonyl)tridecanoate **(65*b*)** (0.322 g, 0.618 mmoles) in AcOEt (6 ml). The mixture was heated at 80°C for 8 hours. After that time, the mixture was filtered under vacuum over Celite, and the solvent was removed under reduced pressure. The residue was dissolved in CH₂Cl₂ (10 ml), and [(methoxycarbonyl)methylene]triphenylphosphorane (0.483 g, 1.44 mmoles) was added. The mixture was stirred at room temperature for 24 hours. After that time, AcOEt (10 ml) and Celite were added, and the solvent was removed under reduced pressure. The product was purified by a chromatographic column (hexane/AcOEt, 5:1), obtaining methyl *rac*-(*Z*,4*R*,5*R*,6*R*,7*S*)-7-(*tert*-butyldimethylsilyloxy)-5,6-(dimethylmethylenedioxy)-4-hydroxy-3,4-bis(methoxycarbonyl)-2-tetradecenoate (**76*b***) (0.270 g, yield 76%), as a colorless oil.
**IR** (NaCl): ν 3471, 2949, 2930, 2857, 1735, 1645, 1461, 1435, 1368, 1254, 1167, 1086, 836, 775 cm⁻¹.
**¹H-NMR** (400 MHz, CDCl₃). δ 6.51 (1H, s, H-2), 4.60 (1H, d, *J* = 6.6 Hz, H-5), 4.50 (1H, s, -OH), 4.04 (1H, dd, *J* = 6.2, 6.6 Hz, H-6), 3.81 (3H, s, -OCH₃), 3.78 (3H, s, - OCH₃), 3.74 (3H, s, -OCH₃), 3.68 (1H, dt, *J* = 6.2, 10.9 Hz, H-7), 1.56 (2H, m, H-8), 1.39 (6H, s, -CH₃), 1.28 (10H, s_{broad}, -CH₂-), 0.90 (12H, m, *tert*-BuSi, -CH₃), 0.10 (6H, m, -CH₃).
**¹³C-NMR** (100 MHz, CDCl₃). δ 170.2, 166.1, 165.0, 145.9, 124.0, 110.6, 79.5, 78.7, 76.3, 73.4, 53.3, 52.1, 51.8, 33.1, 31.6, 29.5, 29.0, 27.0, 26.8, 26.1, 25.6, 23.5, 17.9, 13.8, -4.4, -4.7.
**LRMS(EI):** *m*/*z* 574(M⁺, 0), 559(3), 517(2), 485(2), 409(15), 335(26), 243(100), 215(34).
**E.A.** (C₂₈H₅₀O₁₀Si): Found: C, 58.58, H, 8.87; Calculated: C, 58.51, H, 8.77.

### Example 10: Preparation of methyl rac-(Z,4S,5R,6S)-4,5-epoxy-6-hydroxy-3-(methoxycarbonyl)-2-tridecenoate (82c)

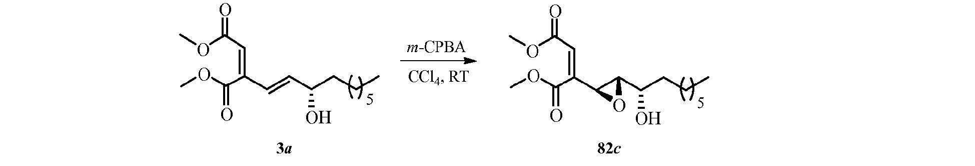

*m*-CPBA (0.101 g, 0.59 mmoles) was added to a solution of methyl *rac-*(2*Z*,4*E*,*S*)-6-hydroxy-3-(methoxycarbonyl)-2,4-tridecadienoate (**3*a***) (0.080 g, 0.26 mmoles) in CCl₄ (2.5 ml). The mixture was stirred at room temperature for 3 days. After that time, the solvent was removed under reduced pressure. The product was purified by a chromatographic column (hexane/AcOEt, 4:1), obtaining (0.080 g, yield 95%) methyl *rac*-(*Z*,4*S*,5*R*,6*S*)-4,5-epoxy-6-hydroxy-3-(methoxycarbonyl)-2-tridecenoate **(82c),** as a colorless oil.
**IR** (NaCl): ν 3473, 2929, 2857, 1730, 1652, 1574, 1437, 1372, 1276, 1204, 1171, 1020, 870 cm⁻¹.
**¹H-NMR** (300 MHz, CDCl₃). δ 6.15 (1H, s, H-2), 3.86 (1H, m), 3.81 (3H, s, -OCH₃), 3.73 (3H, s, -OCH₃), 3.71 (3H, s, -OCH₃), 3.63 (3H, s, -OCH₃), 3.58 (1H, m), 3.05 (1H,

m), 1.63 (2H, m, -CH₂-), 1.25 (10H, s_{broad}, -CH₂-), 0.85 (3H, m, -CH₃).
**¹³C-NMR** (75 MHz, CDCl₃). δ 165.6, 165.6, 165.0, 164.9, 144.5, 144.1, 122.0, 121.8, 70.1, 67.8, 63.9, 63.4, 53.8, 52.6, 52.6, 52.2, 52.1, 34.3, 33.0, 31.6, 29.4, 29.3, 29.0, 25.1,24.9,22.5,14.0.
**LRMS(EI):** *m*/*z* 314(M⁺, 0), 282(5), 237(1), 223(1), 173(10), 156(100), 141(82), 127(10).
**E.A.** (C₁₆H₂₆O₆): Found: C, 61.00, H, 8.41; Calculated: C, 61.13, H, 8.34.

### Example 11: Preparation of methyl rac-(Z,4S,5R,6R)-5,6-(dimethylmethylenedioxy)-4-hydroxy-3,4-bis(methoxycarbonyl)-7-oxo-2-tetradecenoate (110a)

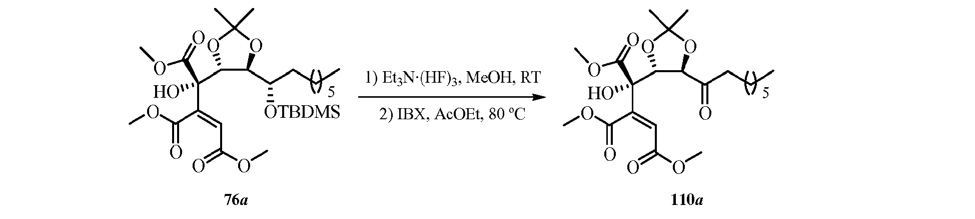

Et₃N·(HF)₃ (0.492 g, 3.05 mmoles) was added to a solution of methyl *rac-*(*Z*,4*S*,5*R*,6*R*,7*S*)-7-(*tert*-butyldimethylsilyloxy)-5,6-(dimethylmethylenedioxy)-4-hydroxy-3,4-bis(methoxycarbonyl)-2-tetradecenoate (**76*a***) (0.220 g, 0.382 mmoles) in MeOH (5 ml). The mixture was stirred at room temperature for 6 days. After that time, AcOEt (10 ml) was added and the mixture washed with H₂O (2 × 5 ml), dried with anhydrous Na₂SO₄, filtered and the solvent was removed under reduced pressure. The residue was dissolved in AcOEt (3.6 ml), and IBX (0.224 g, 0.801 mmoles, 3 eq.) was added. The mixture was heated at 80°C for 7 hours. After that time, the mixture was filtered under vacuum over Celite and the solvent was removed under reduced pressure. The product was purified by a chromatographic column (hexane/AcOEt, 4:1), obtaining (0.110 g, yield 63%) methyl *rac*-(*Z*,4*S*,5*R*,6*R*)-5,6-(dimethylmethylenedioxy)-4-hydroxy-3,4-bis(methoxycarbonyl)-7-oxo-2-tetradecenoate (**110*a***), as a colorless oil. **IR** (NaCl): ν 3477, 2949, 2931, 2854, 1733, 1645, 1455, 1436, 1373, 1201, 1162, 1083, 985, 876 cm⁻¹.
**¹H-NMR** (400 MHz, CDCl₃). δ 6.45 (1H, s, H-2), 5.02 (1H, d, *J* = 4.6 Hz, H-6), 4.51 (1H, d, *J* = 4.6 Hz, H-5), 3.88 (3H, s, -OCH₃), 3.80 (3H, s, -OCH₃), 3.73 (3H, s, - OCH₃), 2.64 (2H, m, H-8), 1.57 (2H, m, H-9), 1.45 (3H, s, -CH₃), 1.25 (13H, s_{broad}, - CH₂-, -CH₃), 0.86 (3H, m, -CH₃).
**¹³C-NMR** (100 MHz, CDCl₃). δ 209.2, 170.7, 166.0, 164.4, 146.2, 122.9, 112.3, 80.6, 79.7, 77.5, 54.2, 52.6, 52.1, 39.2, 31.6, 29.0, 29.0, 26.5, 26.5, 23.1, 22.5, 14.0. **LRMS(EI):** *m*/*z* 458(M⁺, 0), 443(1), 427(0), 399(0), 369(12), 331(7), 313(14), 273(92), 227(32), 127(100).
**E.A.** (C₂₂H₃₄O₁₀): Found: C, 57.70, H, 7.50; Calculated: C, 57.63, H, 7.47.

### Example 12: Preparation of methyl rac-(Z,4R,5R,6R)-5,6-(dimethylmethylenedioxy)-4-hydroxy-3,4-bis(methoxycarbonyl)-7-oxo-2-tetradecenoate (110b)

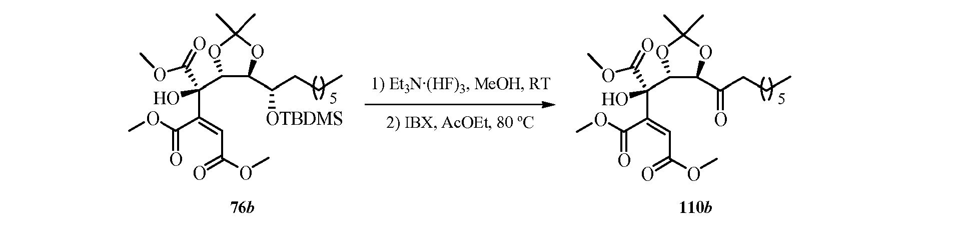

Et₃N·(HF)₃ (0.605 g, 3.75 mmoles) was added to a solution of methyl *rac-*(*Z*,4*R*,5*R*,6*R*,7*S*)-7-(*tert*-butyldimethylsilyloxy)-5,6-(dimethylmethylenedioxy)-4-hydroxy-3,4-bis(methoxycarbonyl)-2-tetradecenoate **(76b)** (0.270 g, 0.469 mmoles) in MeOH (6 ml). The mixture was stirred at room temperature for 6 days. After that time, AcOEt (10 ml) was added and the mixture washed with H₂O (2 × 5 ml), dried with anhydrous Na₂SO₄, filtered and the solvent was removed under reduced pressure. The residue was dissolved in AcOEt (4 ml), and IBX (0.324 g, 1.15 mmoles) was added. The mixture was heated at 80°C for 7 hours. After that time, the mixture was filtered under vacuum over Celite, and the solvent was removed under reduced pressure. The product was purified by a chromatographic column (hexane/AcOEt, 4:1), obtaining (0.160 g, yield 74%) methyl *rac*-(2*Z*,4*R*,5*R*,6*R*)-5,6-(dimethylmethylenedioxy)-4-hydroxy-3,4-bis(methoxycarbonyl)-7-oxo-2-tetradecenoate (**110*b***), as a colorless oil. **IR** (NaCl): ν 3470, 2985, 2949, 2930, 2854, 1732, 1648, 1436, 1373, 1352, 1255, 1166, 1090, 882 cm⁻¹.
**¹H-NMR** (400 MHz, CDCl₃). δ 6.45 (1H, s, H-2), 4.70 (1H, d, *J* = 6.6 Hz, H-6), 4.37 (1H, d, *J* = 6.6 Hz, H-5), 3.96 (1H, s, -OH), 3.83 (3H, s, -OCH₃), 3.81 (3H, s, -OCH₃), 3.74 (3H, s, -OCH₃), 2.66-2.61 (2H, m, H-8), 1.55 (2H, m, H-9), 1.47 (3H, s, -CH₃), 1.38 (3H, s, -CH₃), 1.27 (8H, s_{broad}, -CH₂-), 0.87 (3H, m, -CH₃).
**¹³C-NMR** (100 MHz, CDCl₃). δ 210.6, 169.9, 166.2, 164.7, 146.1, 123.4, 111.7, 80.4, 79.1, 76.3, 53.7, 52.3, 51.9, 38.6, 31.4, 28.8, 28.8, 26.2, 26.08, 22.6, 22.3, 13.8.
**LRMS(EI):** *m*/*z* 459(M⁺+1, 0), 443(2), 399(1), 369(4), 331(10), 313(11), 299(7), 273(50), 255(32), 127(100).
**E.A.** (C₂₂H₃₄O₁₀): Found: C, 57.72, H, 7.55; Calculated: C, 57.63, H, 7.47.

### Example 13: Reaction of methyl rac-(Z,4S,5R,6R)-5,6-(dimethylmethylenedioxy)-4-hydroxy-3,4-bis(methoxycarbonyl)-7-oxo-2-tetradecenoate (110a) with p-TsOH and MeOH

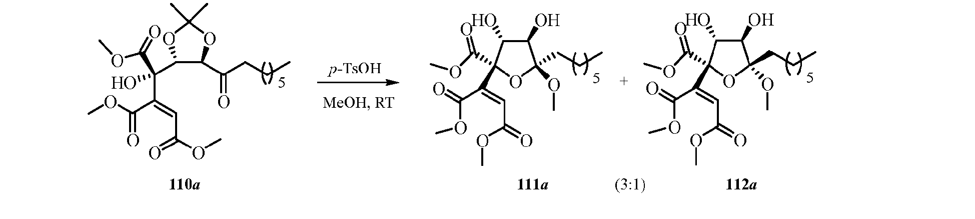

*p*-TsOH (0.008 g, cat.) was added to a solution of methyl *rac*-(*Z*,4*S*,5*R*,6*R*)-5,6-(dimethylmethylenedioxy)-4-hydroxy-3,4-bis(methoxycarbonyl)-7-oxo-2-tetradecenoate **(110*a*)** (0.045 g, 0.098 mmoles) in MeOH (1.3 ml). The mixture was stirred at room temperature for 6 days. After that time, NaHCO₃ (0.03 g) was added and the mixture was stirred for 10 minutes. Then, the mixture was filtered under vacuum over Celite and the solvent was removed under reduced pressure. The product was purified by a chromatographic column (hexane/AcOEt, 3:1), obtaining (0.030 g, yield 71%) methyl *rac*-(*Z*,4*S*,5*R*,6*R*,7*S*)-5,6-dihydroxy-4,7-epoxy-7-methoxy-3,4-bis(methoxycarbonyl)-2-tetradecenoate (**111*a***) and (0.011 g, yield 26%) methyl *rac-*(*Z*,4*S*,5*R*,6*R*,7*R*)-5,6-dihydroxy-4,7-epoxy-7-methoxy-3,4-bis(methoxycarbonyl)-2-tetradecenoate (**112*a***), both as a colorless oil.

### Methyl rac-(Z,4S,5R,6R,7S)-5,6-dihydroxy-4,7-epoxy-7-methoxy-3,4-bis(methoxycarbonyl)-2-tetradecenoate (111a)

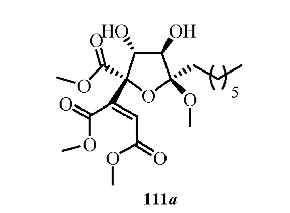

**IR** (NaCl): ν 3466, 2950, 2926, 2852, 1758, 1732, 1642, 1435, 1258, 1169, 1080 cm⁻¹.
**¹H-NMR** (400 MHz, CDCl₃). δ 6.47 (1H, s, H-2), 4.47 (1H, dd, *J* = 4.1, 8.6 Hz, H-5), 4.21 (1H, dd, *J* = 8.6, 10.7 Hz, H-6), 3.81 (3H, s, -OCH₃), 3.74 (3H, s, -OCH₃), 3.73 (3H, s, -OCH₃), 3.64 (1H, d, *J* = 4.1 Hz, -OH), 3.32 (3H, s, -OCH₃), 2.42 (1H, d, *J* = 10.7 Hz, -OH), 1.99 (1H, m, H-8), 1.83 (1H, m, H-8), 1.29 (10H, s_{broad}, -CH₂-), 0.88 (3H, m, -CH₃).
**¹³C-NMR** (100 MHz, CDCl₃). δ 169.5, 167.6, 165.6, 144.0, 122.8, 105.6, 85.0, 82.5, 78.0, 52.9, 52.9, 52.2, 49.1, 32.9, 31.7, 29.7, 29.0, 23.0, 22.6, 14.0.
**LRMS(EI):** *m*/*z* 432(M⁺, 0), 401(0), 383(0), 369(1), 333(6), 323(9), 255(2), 213(73), 181(100), 143 (16), 99(3).
**E.A.** (C₂₀H₃₂O₁₀): Found: C, 55.60, H, 7.55; Calculated: C, 55.55, H, 7.46.

### Methyl rac-(Z,4S,5R,6R,7R)-5,6-dihydroxy-4,7-epoxy-7-methoxy-3,4-bis(methoxycarbonyl)-2-tetradecenoate (112a)

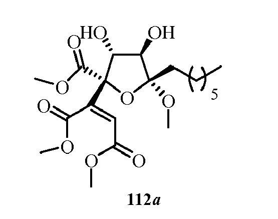

**IR** (NaCl): ν 3491, 2954, 2855, 1732, 1650, 1436, 1351, 1268, 1173, 1076, 1030, 783 cm⁻¹.
**¹H-NMR** (400 MHz, CDCl₃). δ 6.41 (1H, s, H-2), 4.43 (1H, d, *J* = 11.7 Hz, H-5), 4.04 (1H, d, *J* = 8.5 Hz, H-6), 3.95 (1H, d, *J* = 11.7 Hz, -OH), 3.86 (3H, s, -OCH₃), 3.81 (3H, s, -OCH₃), 3.73 (3H, s, -OCH₃), 3.43 (3H, s, -OCH₃), 3.26 (1H, d, *J* = 8.5 Hz, -OH), 1.96 (1H, m, H-8), 1.70 (1H, m, H-8), 1.30 (10H, s_{broad}, -CH₂-), 0.88 (3H, m, -CH₃).
**¹³C-NMR** (100 MHz, CDCl₃). δ 168.2, 168.2, 164.5, 148.3, 122.5, 114.9, 91.5, 86.2, 78.9, 53.1, 53.1, 52.1, 49.2, 31.7, 29.5, 29.1, 28.1, 23.7, 22.6, 14.0.
**LRMS(EI):** *m*/*z* 432(M⁺, 0), 383(0), 333(4), 301(1), 255(1), 213(72), 181(100), 173(39), 99(9).
**E.A.** (C₂₀H₃₂O₁₀): Found: C, 55.62, H, 7.53; Calculated: C, 55.55, H, 7.46.

### Example 14: Reaction of methyl rac-(Z,4S,5R,6R,7S)-5,6-dihydroxy-4,7-epoxy-7-methoxy-3,4-bis(methoxycarbonyl)-2-tetradecenoate (111a) with OsO₄

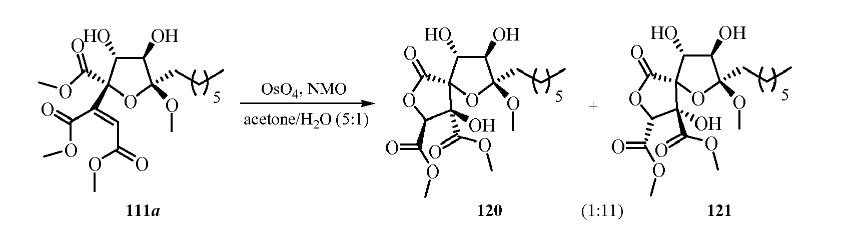

OsO₄ (2.5% in *tert*-BuOH, 0.001 g, 0.004 mmoles) was added to a solution of methyl *rac*-(*Z*,4*S*,5*R*,6*R*,7*S*)-5,6-dihydroxy-4,7-epoxy-7-methoxy-3,4-bis(methoxycarbonyl)-2-tetradecenoate (**111*a***) (0.030 g, 0.069 mmoles) and NMO (0.017 g, 0.15 mmoles) in a 5:1 acetone/H₂O mixture (0.6 ml). The mixture was stirred at room temperature for 6 days. After that time, an aqueous solution of 5% Na₂S₂O₃ (0.5 ml), AcOEt (10 ml) and Celite were added, and the solvent was removed under reduced pressure. The product was purified by a chromatographic column (hexane/AcOEt, 1:1), obtaining (0.005 g, yield 17%) a mixture of methyl *rac*-(2*S*,3*S*,4*R*,5*R*,6*R*,7*S*)-4,7-epoxy-7-methoxy-3-(methoxycarbonyl)-3,5,6-trihydroxytetradecanoate 4,2-carbolactone (120) and methyl *rac*-(2*R*,3*R*,4*R*,5*R*,6*R*,7*S*)-4,7-epoxy-7-methoxy-3-(methoxycarbonyl)-3,5,6-trihydroxytetradecanoate 4,2-carbolactone (121) in a ratio of 1:11, respectively, as a colorless oil.
Methyl *rac*-(2*S*,3*S*,4*R*,5*R*,6*R*,7*S*)-4,7-epoxy-7-methoxy-3-(methoxycarbonyl)-3,5,6-trihydroxytetradecanoate 4,2-carbolactone (120) 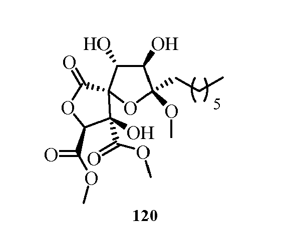 **IR** (KBr): ν 3426, 2922, 2853, 1739, 1636, 1437, 1375, 1077 cm⁻¹.
**¹H-NMR** (400 MHz, CDCl₃). δ 4.98 (1H, s, H-2), 4.77 (1H, d, *J* = 3.0 Hz, -OH), 4.65 (1H, dd, *J* = 3.0, 7.9 Hz, H-5), 4.19 (1H, dd, *J* = 7.9, 10.5 Hz, H-6), 3.91 (3H, s, - OCH₃), 3.86 (3H, s, -OCH₃), 3.82 (1H, s, -OH), 3.19 (3H, s, -OCH₃), 2.36 (1H, d, *J* = 10.5 Hz, -OH), 1.80 (2H, m, H-8), 1.25 (10H, s_{broad}, -CH₂-), 0.87 (3H, t, *J* = 5.1 Hz, - CH₃).
**¹³C-NMR** (100 MHz, CDCl₃). δ 169.9, 169.4, 166.5, 105.7, 85.8, 78.8, 78.5, 75.8, 67.1, 54.3, 53.6, 49.0, 32.1, 31.7, 29.7, 29.0, 23.1, 22.6, 14.0.
**LRMS(EI):** *m*/*z* 434(M⁺, 0), 335(48), 315(16), 290(2), 275(10), 247(52), 229(78), 201(23), 159(100), 101(53).
**E.A.** (C₁₉H₃₀O₁₁): Found: C, 52.60, H, 7.00; Calculated: C, 52.53, H, 6.96.

### methyl rac-(2R,3R,4R,5R,6R,7S)-4,7-epoxy-7-methoxy-3-(methoxycarbonyl)-3,5,6-trihydroxytetradecanoate 4,2-carbolactone (121)

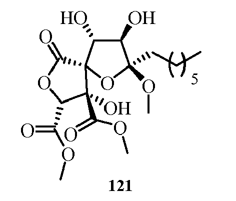

**IR** (KBr): ν 3433, 2953, 2922, 2847, 1804, 1739, 1630, 1437, 1148, 1083, 1055, 800, 499 cm⁻¹.
**¹H-NMR** (400 MHz, CDCl₃). δ 5.43 (1H, s, H-2), 4.28 (2H, m, H-5, H-6), 3.94 (3H, s, - OCH₃), 3.83 (3H, s, -OCH₃), 3.62 (1H, s_{broad}, -OH), 3.26 (3H, s, -OCH₃), 2.36 (1H, d, *J* = 9.0 Hz, -OH), 1.89 (2H, m, H-8), 1.23 (10H, s_{broad}, -CH₂-), 0.89 (3H, m, -CH₃). **¹³C-NMR** (100 MHz, CDCl₃). δ 169.0, 167.5, 165.6, 106.1, 94.9, 80.9, 78.4, 77.7, 77.5, 53.1, 52.9, 49.2, 32.0, 31.6, 29.8, 29.0, 23.2, 22.5, 14.1.
**LRMS(EI):** *m*/*z* 385(1), 355(1), 335(11), 303(1), 290(2), 278(4), 247(15), 217(4), 159(40), 101(15), 83(100).
E.A. (C₁₉H₃₀O₁₁): Found: C, 52.45, H, 6.90; Calculated: C, 52.53, H, 6.96.

### Example 14: Reaction of methyl rac-(Z,4S,5R,6R,7S)-5,6-dihydroxy-4,7-epoxy-7-methoxy-3,4-bis(methoxycarbonyl)-2-tetradecenoate (111a) with RuCl₃/NaIO₄

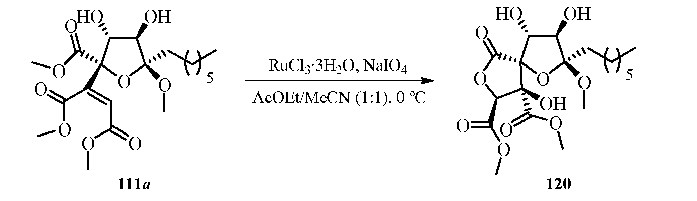

A solution of RuCl₃·3H₂O (0.003 g, 0.014 mmoles) and NaIO₄ (0.018 g, 0.086 mmoles) in H₂O (0.2 ml) was added to a solution of methyl *rac*-(*Z*,4*S*,5*R*,6*R*,7*S*)-5,6-dihydroxy-4,7-epoxy-7-methoxy-3,4-bis(methoxycarbonyl)-2-tetradecenoate (**111*a***)
(0.025 g, 0.057 mmoles) in a 1:1 AcOEt/MeCN mixture (1 ml) at 0°C. The resulting mixture was stirred at 0°C for 5 minutes. After that time, sat. NaHCO₃ (1 ml) and AcOEt (2 ml) were added. The phases were separated, and the aqueous phase was extracted with AcOEt (2 × 1 ml). The organic phase was dried with anhydrous Na₂SO₄, filtered and the solvent was removed under reduced pressure. The product was purified by a chromatographic column (hexane/AcOEt, 1:1), obtaining (0.008 g, yield 32%) methyl *rac*-(2*S*,3*S*,4*R*,5*R*,6*R*,7*S*)-4,7-epoxy-7-methoxy-3-(methoxycarbonyl)-3,5,6-trihydroxytetradecanoate 4,2-carbolactone **(120),** as a colorless oil.
**IR** (KBr): ν 3426, 2922, 2853, 1739, 1636, 1437, 1375, 1077 cm⁻¹.
**¹H-NMR** (400 MHz, CDCl₃). δ 4.98 (1H, s, H-2), 4.77 (1H, d, *J* = 3.0 Hz, -OH), 4.65 (1H, dd, *J* = 3.0, 7.9 Hz, H-5), 4.19 (1H, dd, *J* = 7.9, 10.5 Hz, H-6), 3.91 (3H, s, - OCH₃), 3.86 (3H, s, -OCH₃), 3.82 (1H, s, -OH), 3.19 (3H, s, -OCH₃), 2.36 (1H, d, *J* = 10.5 Hz, -OH), 1.80 (2H, m, H-8), 1.25 (10H, s_{broad}, -CH₂-), 0.87 (3H, t, *J* = 5.1 Hz, - CH₃).
**¹³C-NMR** (100 MHz, CDCl₃). δ 169.9, 169.4, 166.5, 105.7, 85.8, 78.8, 78.5, 75.8, 67.1, 54.3, 53.6, 49.0, 32.1, 31.7, 29.7, 29.0, 23.1, 22.6, 14.0.
**LRMS(EI):** *m*/*z* 434(M⁺, 0), 335(48), 315(16), 290(2), 275(10), 247(52), 229(78), 201(23), 159(100), 101(53).
**E.A.** (C₁₉H₃₀O₁₁): Found: C, 52.60, H, 7.00; Calculated: C, 52.53, H, 6.96.

### Example 15: Reaction of methyl rac-(Z,4S,5R,6R,7R)-5,6-dihydroxy-4,7-epoxy-7-methoxy-3,4-bis(methoxycarbonyl)-2-tetradecenoate (112a) with RuCl₃/NaIO₄

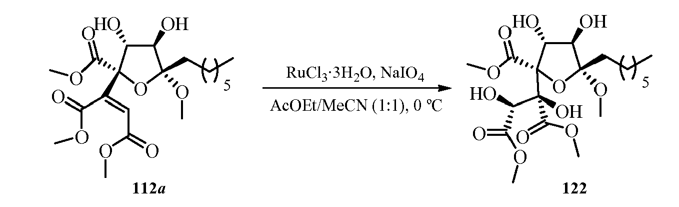

A solution of RuCl₃·3H₂O (0.002 g, 0.010 mmoles) and NaIO₄ (0.013 g, 0.062 mmoles) in H₂O (0.2 ml) was added to a solution of methyl *rac*-(*Z*,4*S*,5*R*,6*R*,7*R*)-5,6-dihydroxy-4,7-epoxy-7-methoxy-3,4-bis(methoxycarbonyl)-2-tetradecenoate (**112*a***) (0.018 g, 0.041 mmoles) in a 1:1 AcOEt/MeCN mixture (1 ml) at 0°C. The mixture was stirred at 0°C for 5 minutes. After that time, an aqueous solution of 10% Na₂S₂O₃ (0.2 ml), H₂O (2 ml) and AcOEt (2 ml) were added. The phases were separated, and the aqueous phase was extracted with AcOEt (2 × 1 ml). The organic phase was dried with anhydrous Na₂SO₄, filtered and the solvent was removed under reduced pressure. The product was purified by a chromatographic column (hexane/AcOEt, 1:2), obtaining (0.014 g, yield 74%) methyl *rac*-(2*S*,3*S*,4*S*,5*R*,6*R*,7*R*)-4,7-epoxy-7-methoxy-3,4-bis(methoxycarbonyl)-2,3,5,6-tetrahydroxytetradecanoate **(122),** as a colorless oil.
**IR** (NaCl): ν 3435, 2957, 2926, 2852, 1643, 1438, 1074 cm⁻¹.
**¹H-NMR** (400 MHz, CDCl₃). δ 5.25 (1H, d, *J* = 6.7 Hz, H-2), 5.06 (1H, d, *J* = 10.1 Hz, H-5), 4.51 (1H, s, -OH), 4.18 (1H, d, *J* = 10.7 Hz, -OH), 3.92 (1H, d, *J* = 10.7 Hz, H-6), 3.85 (3H, s, -OCH₃), 3.83 (3H, s, -OCH₃), 3.75 (3H, s, -OCH₃), 3.59 (1H, d, *J* = 6.7 Hz, -OH), 3.36 (3H, s, -OCH₃), 3.25 (1H, d, *J* = 10.1 Hz, -OH), 1.85 (1H, m, H-8), 1.66 (1H, m, H-8), 1.27 (10H, s_{broad}, -CH₂-), 0.87 (3H, m, -CH₃).
**¹³C-NMR** (100 MHz, CDCl₃). δ 170.8, 170.8, 170.7, 113.7, 79.7, 79.4, 78.8, 78.3, 73.8, 53.9, 52.9, 52.4, 49.5, 31.8, 29.7, 29.1, 28.1, 23.4, 22.6, 14.0.
**LRMS(EI):** *m*/*z* 466(M⁺, 0), 385(2), 375(3), 367(4), 335(11), 315(6), 275(4), 247(15), 229(26), 185(22), 159(100), 127(49).
**E.A.** (C₂₀H₃₄O₁₂): Found: C, 51.65, H, 7.20; Calculated: C, 51.50, H, 7.35.

### Example 16: Preparation of rac-(1R,3S,4S,5S,6R,7R)-1-heptyl-3,4,5-tris(methoxycarbonyl)-4,6,7-trihydroxy-2,8-dioxabicyclo[3.2.1]octane (3)

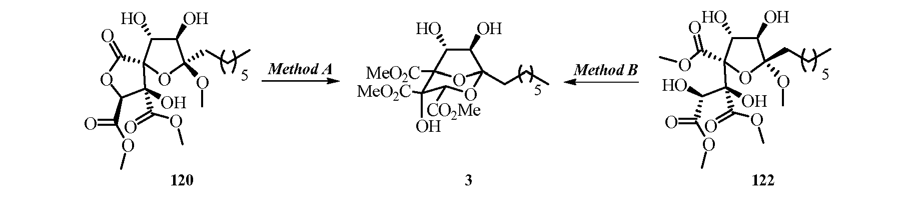

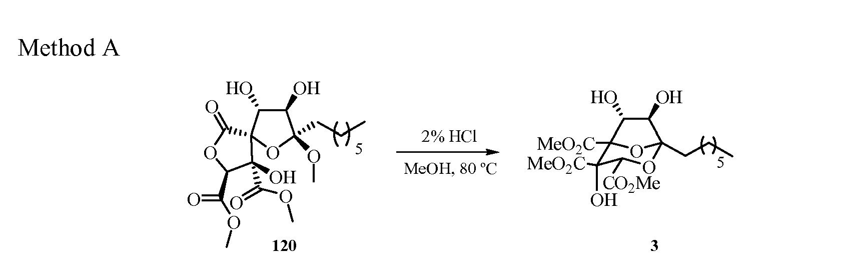

A solution of methyl *rac*-(2*S*,3*S*,4*R*,5*R*,6*R*,7*S*)-4,2-carbolactone-4,7-epoxy-7-methoxy-3-(methoxycarbonyl)-3,5,6-trihydroxy-2-tetradecenoate **(120)** (0.008 g, 0.018 mmoles) in HCl (2% in MeOH, 2.37 ml) was heated in a Kimble at 80°C for 30 hours. After that time, the solvent was removed under reduced pressure. The product was purified by a chromatographic column (hexane/AcOEt, 1:1), obtaining (0.006 g, yield 75%) *rac*-(1*R*,3*S*,4*S*,5*S*,6*R*,7*R*)-1-heptyl-3,4,5-tris(methoxycarbonyl)-4,6,7-trihydroxy-2,8-dioxabicyclo[3.2.1]octane **(3),** as a white solid.

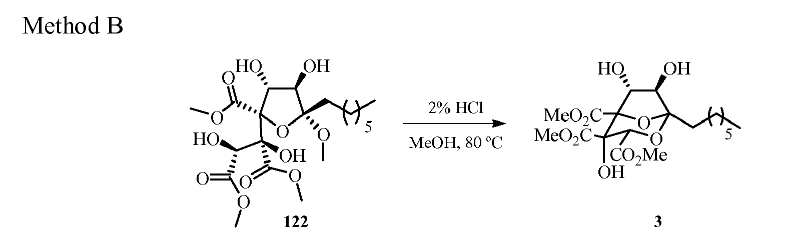

A solution of methyl *rac*-(2*S*,3*S*,4*S*,5*R*,6*R*,7*R*)-4,7-epoxy-7-methoxy-3,4-bis(methoxycarbonyl)-2,3,5,6-tetrahydroxy-2-tetradecanoate **(122)** (0.026 g, 0.055 mmoles) in HCl (2% in MeOH, 2.5 ml) was heated in a Kimble at 80°C for 30 hours.⁷³ After that time, the solvent was removed under reduced pressure. The product was purified by a chromatographic column (hexane/AcOEt, 1:1), obtaining (0.019 g, yield 73%) *rac*-(1*R*,3*S*,4*S*,5*S*,6*R*,7*R*)-1-heptyl-3,4,5-tris(methoxycarbonyl)-4,6,7-trihydroxy-2,8-dioxabicyclo[3.2.1]octane **(3),** as a white solid.

### rac-(1R,3S,4S,5S,6R,7R)-1-Heptyl-3,4,5-tris(methoxycarbonyl)-4,6,7-trihydroxy-2,8-dioxabicyclo[3.2.1]octane (3)

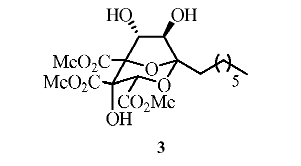

**m.p.:** > 220°C.
**¹H-NMR** (400 MHz, CDCl₃). δ 5.09 (1H, s, H-3), 5.08 (1H, dd, *J* = 2.4, 5.4 Hz, H-6), 4.14 (1H, dd, *J* = 2.4, 3.7 Hz, H-7), 3.84 (3H, s, -OCH₃), 3.74 (3H, s, -OCH₃), 3.68 (3H, s, -OCH₃), 3.67 (1H, s, -OH), 2.5 (1H, d, *J* = 5.4 Hz, -OH), 2.40 (1H, d, *J* = 3.7 Hz, - OH), 1.89 (2H, m, H-1'), 1.21 (10H, s_{broad}, -CH₂-), 0.81 (3H, m, -CH₃).
**¹³C-NMR** (100 MHz, CDCl₃). δ 169.7, 167.0, 166.9, 106.3, 91.4, 82.2, 78.4, 75.4, 74.6, 53.6, 53.0, 52.6, 35.5, 31.7, 29.5, 29.0, 22.7, 22.6, 14.0.
**LRMS(EI):** *m*/*z* 434(M⁺, 0), 336(0), 314(0), 278(0), 243(0), 219(1), 149(3), 83(100). **E.A.** (C₁₉H₃₀O₁₁): Found: C, 52.50, H, 6.90; Calculated: C, 52.53, H, 6.96.

## Claims

1. A process for obtaining a compound of formula (I), its enantiomers or mixtures thereof 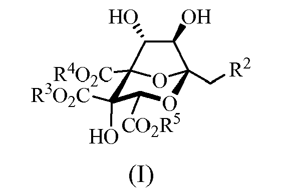 wherein
R² is selected from the group consisting of C₁-C₂₀ alkyl and C₁-C₂₀ alkenyl, which are unsubstituted or substituted in any position with at least one group which is selected from the group consisting of C₁-C₄ alkyl, C₁-C₃ alkylidene, C₁-C₃ alkylcarboxyhydroxyl, hydroxyl and protected hydroxyl; and/or substituted with a group in the end position of the chain which is selected from the group consisting of C₆-C₁₀ aryl, mono- or bicyclic heteroaryl with 5- or 6-members in each ring, which can be unsubstituted or substituted with at least one group which is selected from the group formed by C₁-C₃ alkyl, or halogen; and
R³, R⁴ and R⁵ are independently selected from the group of C₁-C₃ alkyls;
**characterized in that** it comprises reacting in acidic medium a compound of formula (II), its enantiomers or mixtures thereof, or a compound of formula (III), its enantiomers or mixtures thereof, or a mixture of compounds of formula (II) and (III) 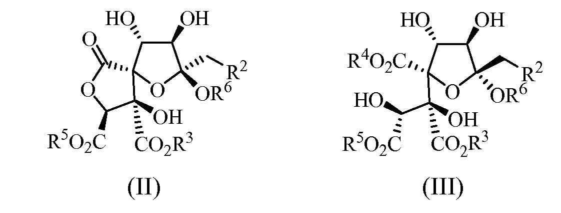 wherein
R², R³, R⁴ and R⁵ are as defined above; and
R⁶ is a C₁-C₃ alkyl group.

2. The process according to claim 1, wherein R³ and R⁴ are identical.

3. The process according to any of the previous claims, wherein R³, R⁴ and R⁵ are identical, preferably methyl.

4. A compound of formula (II), its enantiomers or mixtures thereof, as defined in any of claims 1 to 3.

5. The compound of formula (III), its enantiomers or mixtures thereof, as defined in any of claims 1 to 3.

6. A process for the synthesis of a compound of formula (II), its enantiomers or mixtures thereof, or of a compound of formula (III), its enantiomers or mixtures thereof, as defined in any of claims 1 to 3, **characterized in that** it comprises the dihydroxylation of a compound of formula (IV), its enantiomers or mixtures thereof 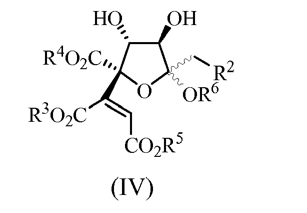 wherein
R², R³, R⁴ and R⁵ are as they have been defined in any of claims 1 to 3; and
R⁶ is a C₁-C₃ alkyl group.

7. The process according to claim 6, wherein the dihydroxylation takes place in the presence of OsO₄ or of RuCl₃.

8. The process according to any of claims 6 and 7, wherein a compound of formula (IVa), its enantiomers or mixtures thereof, is dihydroxylated in the presence of RuCl₃ to yield a compound of formula (II), its enantiomers or mixtures thereof 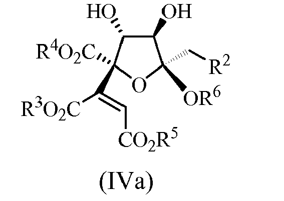 wherein
R², R³, R⁴ and R⁵ are as they have been defined in any of claims 1 to 3; and
R⁶ is a C₁-C₃ alkyl group.

9. The process according to any of claims 6 and 7, wherein a compound of formula (IVb), its enantiomers or mixtures thereof, is dihydroxylated in the presence of RuCl₃ to yield a compound of formula (III), its enantiomers or mixtures thereof 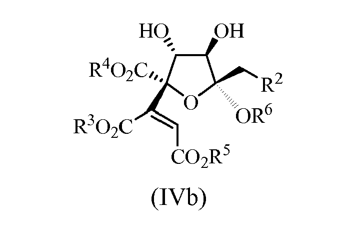 wherein
R², R³, R⁴ and R⁵ are as they have been defined in any of claims 1 to 3; and
R⁶ is a C₁-C₃ alkyl group.

10. A compound of formula (IV), (IVa) or (IVb), its stereoisomers, especially its enantiomers, or mixtures thereof as defined in any of claims 6 to 9.

11. A process for the synthesis of a compound of formula (IV), its stereoisomers, especially its enantiomers, or mixtures thereof, comprising the treatment in acidic medium of a compound of formula (V), its stereoisomers, especially its enantiomers, or mixtures thereof 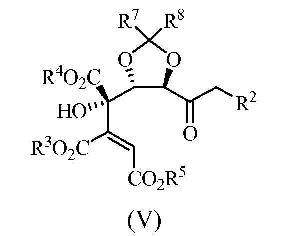 wherein
R², R³, R⁴ and R⁵ are as they have been defined in any of claims 1 to 3; and each of R⁷ and R⁸ is independently selected from the group consisting of hydrogen, C₁-C₄ alkyl and C₆-C₁₀ aryl; or R⁷ and R⁸, together with the carbon atom to which they are attached, form a C₂-C₇ alkylidene group.

12. The process according to claim 11, wherein a compound of formula (V), its stereoisomers, especially its enantiomers, or mixtures thereof, reacts in the presence of p-toluenesulfonic acid.

13. A compound of formula (V), its stereoisomers, especially its enantiomers, or mixtures thereof, as defined in claim 11.

14. A process for the synthesis of a compound of formula (V), its stereoisomers, especially its enantiomers, or mixtures thereof, **characterized in that** it comprises
(i) the removal of the trialkylsilyl group from a compound of formula (VII), its stereoisomers, especially its enantiomers, or mixtures thereof, to yield a compound of formula (VI), its stereoisomers, especially its enantiomers, or mixtures thereof 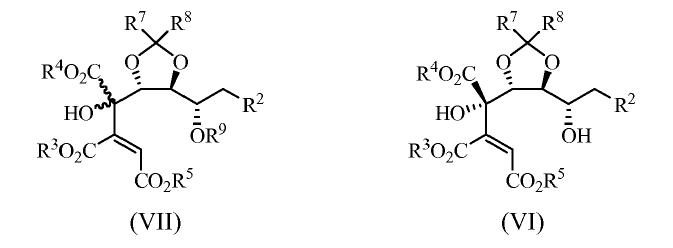 wherein
R², R³ and R⁵ are as they have been defined in any of claims 1 to 3;
R⁶ is a C₁-C₃ alkyl group;
each of R⁷ and R⁸ is independently selected from the group consisting of hydrogen, C₁-C₄ alkyl and C₆-C₁₀ aryl; or R⁷ and R⁸, together with the carbon atom to which they are attached, form a C₂-C₇ alkylidene group; and
R⁹ is a trialkylsilyl group;
and
(ii) the oxidation of the hydroxyl group of a compound of formula (VI), its stereoisomers, especially its enantiomers, or mixtures thereof.

15. The process according to claim 14, wherein the oxidation is performed in the presence of PCC or IBX.

16. A compound of formula (VI), its stereoisomers, especially its enantiomers, or mixtures thereof, as defined in claim 14.

17. A compound of formula (VII), its stereoisomers, especially its enantiomers, or mixtures thereof, as defined in claim 14.

18. A process for the synthesis of a compound of formula (VII), as defined in claim 17, its stereoisomers, especially its enantiomers, or mixtures thereof, which comprises reacting a compound of formula (VIII), its enantiomers, or mixtures thereof, with a compound of formula (XX) 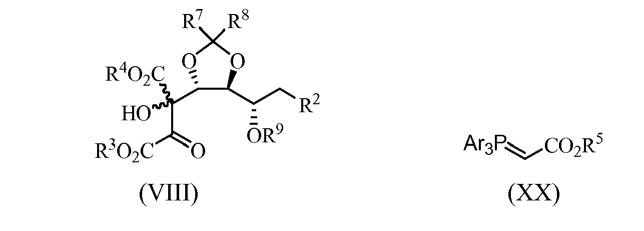 wherein
R², R³, R⁴ and R⁵ are as they have been defined in any of claims 1 to 3; and each of R⁷ and R⁸ is independently selected from the group consisting of hydrogen, C₁-C₄ alkyl and C₆-C₁₀ aryl; or R⁷ and R⁸, together with the carbon atom to which they are attached, form a C₂-C₇ alkylidene group;
R⁹ is a trialkylsilyl group; and
each of the Ar groups is independently selected from among C₆-C₁₀ aryl groups.

19. The process according to claim 18, wherein said compound of formula (VIII), its enantiomers, or mixtures thereof, is obtained by oxidizing, preferably in the presence of IBX, a compound of formula (IX), its enantiomers, or mixtures thereof 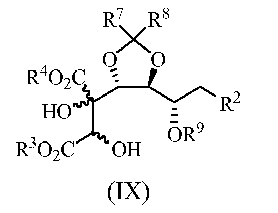 wherein
R², R³ and R⁴ are as they have been defined in any of claims 1 to 3;
each of R⁷ and R⁸ is independently selected from the group consisting of hydrogen, C₁-C₄ alkyl and C₆-C₁₀ aryl; or R⁷ and R⁸, together with the carbon atom to which they are attached, form a C₂-C₇ alkylidene group; and
R⁹ is a trialkylsilyl group.

20. The process according to claims 18 and 19, wherein the transformation of a compound of formula (IX), its enantiomers, or mixtures thereof, into a compound of formula (VII), its stereoisomers, especially its enantiomers, or mixtures thereof, is performed without isolating said compound of formula (VIII).

21. A compound of formula (VIII), its enantiomers, or mixtures thereof, as defined in claim 18.

22. A compound of formula (IX), its enantiomers, or mixtures thereof, as defined in claim 19.

23. A process for the synthesis of a compound of formula (IX), its enantiomers, or mixtures thereof, **characterized in that** it comprises the dihydroxylation, preferably in the presence of OsO₄, of a compound of formula (X), its enantiomers, or mixtures thereof 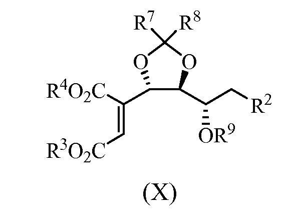 wherein
R², R³ and R⁴ are as they have been defined in any of claims 1 to 3;
each of R⁷ and R⁸ is independently selected from the group consisting of hydrogen, C₁-C₄ alkyl and C₆-C₁₀ aryl; or R⁷ and R⁸, together with the carbon atom to which they are attached, form a C₂-C₇ alkylidene group; and
R⁹ is a trialkylsilyl group.

24. A compound of formula (X), its enantiomers, or mixtures thereof, as defined in claim 23.

25. A process for the synthesis of a compound of formula (X), its enantiomers, or mixtures thereof, **characterized in that** it comprises the acetalization or hemiacetalization, of a compound of formula (XI), its enantiomers, or mixtures thereof 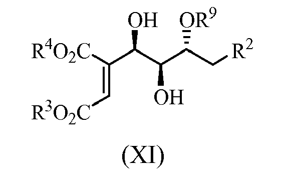 in the presence of a compound of formula (R₇)(R₈)C=O or hydrates or aryl or alkyl acetals or hemiacetals thereof
wherein
R², R³ and R⁴ are as they have been defined in any of claims 1 to 3; and
each of R⁷ and R⁸ is independently selected from the group consisting of hydrogen, C₁-C₄ alkyl and C₆-C₁₀ aryl; or R⁷ and R⁸, together with the carbon atom to which they are attached, form a C₂-C₇ alkylidene group; and
R⁹ is a trialkylsilyl group.

26. A compound of formula (XI), its enantiomers, or mixtures thereof, as defined in claim 25.

27. A process for the synthesis of a compound of formula (XI), its enantiomers, or mixtures thereof, **characterized in that** it comprises the following steps (i) reacting a compound of formula (XXI) in the presence of a compound of formula PY₃, and subsequently adding a compound of formula (XVI), its stereoisomers or mixtures thereof, to yield a compound of formula (XV), its stereoisomers or mixtures thereof 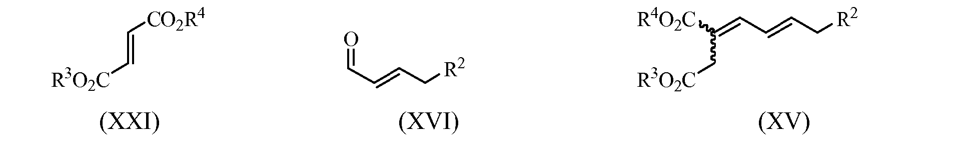 wherein
R², R³ and R⁴ are as they have been defined in any of claims 1 to 3;
each of the groups Y is independently selected from among C₆-C₁₀ aryl groups or C₁-C₆ alkyl groups;
(ii) epoxidizing, preferably with meta-chloroperbenzoic acid (m-CPBA), said compound of formula (XV), to obtain a compound of formula (XIV), its stereoisomers, especially its enantiomers, or mixtures thereof, 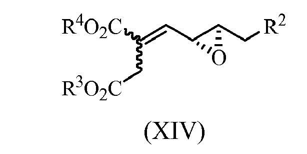 wherein
R², R³ and R⁴ are as defined above;
(iii) isomerizing in the presence of a base, preferably DBU, said compound of formula (XIV), to obtain a compound of formula (XIII), its stereoisomers, especially its enantiomers, or mixtures thereof, 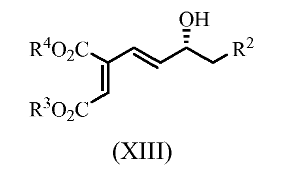 wherein
R², R³ and R⁴ are as defined above;
(iv) introducing a trialkylsilyl group into said compound of formula (XIII) to obtain a compound of formula (XII), its stereoisomers, especially its enantiomers, or mixtures thereof 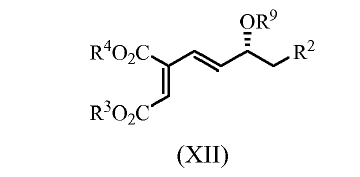 wherein
R², R³ and R⁴ are as defined above; and
R⁹ is a trialkylsilyl group;
and
(v) dihydroxylating said compound of formula (XII).

28. A compound of formula (XV), as defined in claim 27, its stereoisomers, or mixtures thereof.

29. A compound of formula (XIV), as defined in claim 27, its stereoisomers, especially enantiomers, or mixtures thereof.

30. A compound of formula (XIII), as defined in claim 27, its stereoisomers, especially enantiomers, or mixtures thereof.

31. A compound of formula (XII), as defined in claim 27, its stereoisomers, especially enantiomers, or mixtures thereof.

32. A process for preparing zaragozic acid and derivatives thereof of formula (XXVI), their stereoisomers, especially enantiomers, or mixtures thereof, **characterized in that** it comprises the following steps
(i) reacting in acidic medium a compound of formula (II), its enantiomers or mixtures thereof, or a compound of formula (III), its enantiomers or mixtures thereof, or a mixture of compounds of formula (II) and (III), as defined in any of claims 1 to 3; to obtain a compound of formula (I), its enantiomers or mixtures thereof, as defined in any of claims 1 to 3;
(ii) hydrolyzing in basic medium the ester groups of said compound of formula (I), its enantiomers or mixtures thereof, to provide a compound of formula (XXV), its enantiomers or mixtures thereof 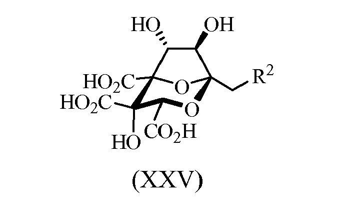 wherein
R² is as defined in claim 1;
and
(iii) reacting said compound of formula (XXV), its enantiomers or mixtures thereof, with a compound of formula (XXII) in the presence of a base, to obtain a compound of formula (XXVI), its enantiomers or mixtures thereof 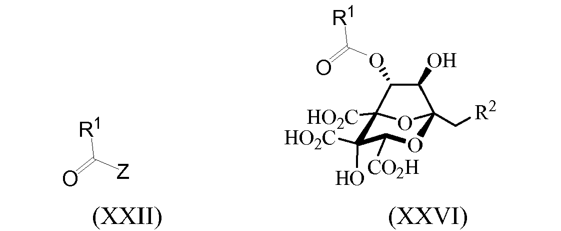 wherein
R¹ is selected from the group consisting of C₁-C₂₀ alkyl or C₁-C₂₀ alkenyl, which are unsubstituted or substituted with at least one group which is selected from the group consisting of C₁-C₄ alkyl; and/or a group in the end position which is selected from the group consisting of C₆-C₁₀ aryl;
Z is selected from the group consisting of hydroxyl and alkoxyl; and
R² is as defined in claim 1.

33. Use of a compound of formula (I), (II), (III), (IV), (V), (VI), (VII), (VIIa), (VIIb), (VIII), (VIIa), (VIIIb), (IX), (IXa), (IXb), (X), (XI), (XII), (XIII), (XIV), (XV) and/or (XVI), its stereoisomers, especially enantiomers, or mixtures thereof, for the synthesis of zaragozic acid and derivatives thereof of formula (XXVI), their stereoisomers, especially enantiomers, or mixtures thereof.
